# EUROPEAN PATENT APPLICATION

(11) **EP 4 815 525 A1**
(43) Date of publication of application: **30.09.2026**
(21) Application number: 25880927.6
(22) Date of filing: 23.12.2025
(51) Int. Cl.: H04W 4/90, G08B 25/00, G08B 25/01, H04W 4/16, G06F 16/34, G10L 15/26, A61B 5/00, H04W 84/06

(54) **SATELLITE COMMUNICATION SYSTEM**

(30) Priority: 23.12.2024 KR 20240193714
(71) Applicant: LG ELECTRONICS INC., Yeongdeungpo-gu Seoul 07336 (KR)
(72) Inventor: IM, Sehyeok, Seoul 07796 (KR); KIM, Pilsang, Seoul 07796 (KR); KIM, Sujin, Seoul 07796 (KR); PARK, Taegun, Seoul 07796 (KR)
(74) Representative: Schott, Jakob Valentin
(86) International application number: PCT/KR2025/022569
(87) International publication number: WO 2026/142260

(57) **Abstract**

Provided is a satellite communication method including acquiring at least one of vehicle information and driver information based on at least one of collected voice data, sensor data, and user input data, classifying a driver context as at least one of a plurality of context types based on the acquired information, generating a command message based on the context type and the acquired information, performing data lightweighting to reduce a size of the command message, and transmitting the lightweighted command message via a satellite.

## Description

### Technical Field

The disclosure relates to a satellite communication system. More specifically, one or more embodiments relate to a satellite communication system and method designed to provide data transmission and intelligent emergency response services optimized for vehicle and driver statuses by interworking an on-device AI of a user terminal with a context-aware AI of a server in a non-terrestrial network (NTN) environment.

### Background Art

Due to recent developments in communication technology, non-terrestrial network (NTN) technologies are attracting attention to provide uninterrupted connectivity in dead zones, sea, air, and the like where terrestrial network (TN) does not reach. In particular, communication services utilizing low-earth orbit (LEO) or geostationary orbit (GEO) satellites are becoming essential communication methods in emergency rescue requests or disaster situations.

However, these satellite communication technologies have several limitations as follows.

First, there is the issue of accessibility due to high cost and bandwidth constraints of satellite communications. Satellite-based voice calls typically cost significantly more than terrestrial networks, have slower data transmission speeds, and have limited bandwidth. Accordingly, existing systems rely on a method of simply transmitting text messages or transmitting low-quality voice data as it is. This method not only increases communication costs by failing to efficiently reduce the size of data packets, but also risks missing the golden time by causing transmission failures or delays in case of network congestion during emergency contexts.

Second, there is the issue of inaccurate context awareness due to simple data transmission. Related art emergency rescue systems only transmit fragmented information, such as user-entered text or GPS coordinates. However, in an actual accident site, a driver is often confused and has difficulty in inputting an exact context, or loses consciousness and is unable to operate a vehicle. Sensor values or text alone fail to provide a comprehensive view of the driver's psychological state, specific injury severity, or ambient noise level, which causes rescue centers to face limitations in executing appropriate initial interventions to the context.

Third, there is limited bidirectional communication and lack of user experience (UX). Existing satellite terminals are primarily focused on one-way rescue requests, making bidirectional interaction difficult, such as receiving feedback after a rescue request or receiving specific action instructions (guides) before the rescue team arrives. When an accident occurs while traveling abroad, communication with local rescue teams may be impossible due to language barriers, and even when trying to use convenience services, such as restaurant reservations or general inquiries via satellite networks, voice calls may not be supported or service use may be limited due to the burden of fees.

Therefore, there is a growing technological demand for intelligent satellite communication systems that can dramatically reduce data packets and increase communication efficiency even in bandwidth-limited satellite communication environments, while utilizing AI technologies to accurately recognize the driver's context and provide active bidirectional guides. In particular, there is a growing need for integrated solutions that can reduce communication costs through text-to-speech (STT/TTS) and compression technologies, and flexibly respond to both emergency and general contexts through server-based AI assistants.

### Disclosure of Invention

### Technical Problem

The disclosure aims to solve the aforementioned problems and other drawbacks, and an aspect of the disclosure is to provide a satellite communication system for optimizing data by utilizing on-device AI in contexts where communication resources are limited, such as a non-terrestrial network (NTN) environment, and providing an intelligent emergency response service by interworking with an AI assistant of a server, and an operating method thereof.

Another aspect of the disclosure is to minimize data packets and maximize transmission efficiency by converting voice data into text in a terminal and selectively extracting, compressing, and transmitting only necessary data according to a context type, to overcome high costs and bandwidth constraints of satellite communications.

Another aspect of the disclosure is to provide a satellite communication method capable of performing accurate context awareness by synthetically analyzing a driver's voice, psychological state, and location information through an on-device AI, and accurately determining an emergency urgency level based on the context awareness, beyond the limitations of the related art systems that rely merely on text or sensor inputs.

Another aspect of the disclosure is to provide uninterrupted bidirectional communication and improved user experience (UX) by utilizing server-based conversational AI and large language models (LLMs) even in text-based satellite communication environments to provide users with context-specific customized response guides or to perform voice calls to emergency centers on behalf of the users.

### Solution to Problem

A satellite communication method according to the disclosure may include acquiring at least one of vehicle information and driver information based on at least one of collected voice data, sensor data, and user input data, classifying a driver context as at least one of a plurality of context types based on the acquired information, generating a command message based on the context type and the acquired information, performing data lightweighting to reduce a size of the command message, and transmitting the lightweighted command message via a satellite.

According to an embodiment, the method may further include extracting necessary data associated with the context type, and adding the necessary data to the command message.

According to an embodiment, the method may further include determining whether an emergency context exists or an emergency urgency level based on the acquired information, and the context type may be classified based on whether the emergency context exists or the emergency urgency level.

According to an embodiment, the context type may include at least one of emergency call, emergency response guide, telephone reservation, and information search.

According to an embodiment, the determining the emergency urgency level may include analyzing acoustic characteristics of the voice data to derive a driver's psychology index, and determining the emergency urgency level by reflecting the driver's psychology index.

According to an embodiment, the determining the emergency urgency level may include acquiring location information from the sensor data to calculate a risk weight corresponding to the location information, and determining the emergency urgency level by reflecting the risk weight.

According to an embodiment, the method may further include converting the voice data into text data using a speech-to-text (STT) model, and adding the text data to the command message.

According to an embodiment, the method may further include determining whether the text data and the sensor data are different from each other, and performing content correction on the text data by applying priority to the sensor data when the text data and the sensor data are different from each other.

According to an embodiment, the method may further include receiving a response message to the transmitted command message from the server via the satellite, and performing a response operation associated with the received response message.

According to an embodiment, the necessary data may include at least one of where a crash has occurred, vehicle identification information, location information, vehicle speed, and occupant information when the context type is an emergency call or emergency response guide, and may include at least one of location information, user information, vehicle information, and service request information when the context type is a telephone reservation or information search.

According to an embodiment, the performing the data lightweighting may include performing summarization or compression on the command message, and a summarization level or compression rate for the command message may be applied differently depending on at least one of the context type and satellite communication link quality.

According to an embodiment, the command message may be compressed at a first compression ratio when the context type is an emergency call or emergency response guide or when the satellite communication link quality is below a threshold, and the command message may be compressed at a second compression rate lower than the first compression rate when the context type is a telephone reservation or information search and the satellite communication link quality is higher than or equal to the threshold.

According to an embodiment, the method may further include decompressing the received command message by a server which has received the command message, analyzing the command message to identify the context type included in the command message, and summarizing or compressing a response message including a result of performing a context-specific operation according to the identified context type and transmitting the summarized or compressed response message to a user terminal via the satellite.

According to an embodiment, the method may further include, when the identified context type is an emergency call, analyzing the command message to acquire rescue request information, connecting an emergency call with an emergency response center by activating server-based interactive artificial intelligence (AI), performing a voice-based conversation (call conversation) with the emergency response center based on the rescue request information, and generating the response message to include feedback information comprising dispatch information or response results of an emergency responder.

According to an embodiment, the method may further include, when the identified context type is an emergency response guide, structuring a large language model (LLM) request (LLM REQUEST) requesting a context analysis and transmitting the LLM request to the LLM, in response to the transmission, receiving an LLM response (LLM RESPONSE) comprising a context analysis result from the LLM, and analyzing and restructuring the LLM response.

According to an embodiment, the method may further include, when the identified context type is a telephone reservation, analyzing the command message to acquire reservation information, activating server-based conversational AI to connect a call with a reservation target included in the reservation information, performing a voice-based conversation with the reservation target based on the reservation information, and generating the response message to include feedback information including whether the reservation has been successful or reservation details.

According to an embodiment, the method may further include, when the identified context type is information search, acquiring search information by analyzing the command message, generating a search query based on the search information and performing an information search by interworking with an external search engine, performing text summarization by extracting key information that matches a user intent from result data of the information search, and generating the response message to include the summarized text data.

According to an embodiment, the structuring the LLM request requesting the context analysis may include selecting a context-specific prompt model based on at least one of the context type included in the command message, necessary data, and text data, creating a prompt set or context-specific query using the selected prompt model, and structuring an LLM request including the prompt set or the context-specific query.

According to an embodiment, the method may further include acquiring a plurality of step-by-step guide texts based on the restructured LLM response, and generating a response message including the plurality of step-by-step guide texts, and the response message may further include a text-to-speech (TTS) command that activates an on-device artificial intelligence (AI) model to instruct the plurality of step-by-step guide texts via voice.

According to an embodiment, the method may further include re-determining whether the driver context is an emergency call context based on the restructured LLM response, wherein the method further comprises, when the driver context is determined as the emergency call context according to a result of the re-determination, connecting an emergency call with an emergency response center by activating server-based conversational AI, performing a voice-based conversation with the emergency response center based on the restructured LLM response, and generating the response message to include feedback information including dispatch information or response results of an emergency responder.

According to an embodiment, the method may further include, in the performing the response operation, when the response message includes a TTS command, converting a first guide text, among the plurality of step-by-step guide texts included in the response message, into voice and outputting the converted voice, detecting the user's response input to the output voice, and sequentially performing an operation of converting and outputting a second guide text into voice when the response input is detected.

According to an embodiment, the performing the response operation may include identifying a country code by acquiring at least one of location information and phone number information, determining a target language based on the identified country code, and automatically translating the received response message into the determined target language and outputting the translated response message.

A vehicle configured to perform satellite communication according to the disclosure may include a communication module configured to transmit and receive data to and from a server by interworking with a non-terrestrial network (NTN) via a satellite, and a processor operably connected to the communication module, and the processor may acquire at least one of vehicle information and driver information based on at least one of collected voice data, sensor data, and user input data, classify a driver context as at least one of a plurality of context types based on the acquired information, generate a command message based on the context type and the acquired information, perform data lightweighting to reduce a size of the command message, and transmit the lightweighted command message via the satellite.

A computer-readable storage medium according to the disclosure may store at least one computer program including instructions that, when executed by at least one processor, cause the at least one processor to perform operations, and the operations may include acquiring at least one of vehicle information and driver information based on at least one of collected voice data, sensor data, and user input data, classifying a driver context as at least one of a plurality of context types based on the acquired information, generating a command message based on the context type and the acquired information, performing data lightweighting to reduce a size of the command message, and transmitting the lightweighted command message via a satellite.

### Advantageous Effects of Invention

According to the disclosure, by utilizing on-device AI within a terminal in a non-terrestrial network (NTN) environment to convert voice data into text (STT), and selectively extracting, compressing, and transmitting only necessary data according to a context type, the high cost issue and bandwidth constraints of the related art satellite communication can be effectively solved. Accordingly, communication charges can be reduced by minimizing the size of data packets, and data transmission success rate and speed can be dramatically improved even in network congestion situations.

According to the disclosure, unlike the related art methods of simply transmitting text or sensor values, the driver's voice features (tone, intensity, and the like), psychological state, and location information can be comprehensively analyzed through the on-device AI model, thereby enabling precise judgment of whether an accident has occurred and an urgency level. This can reduce false reports and accurately recognize actual emergency contexts, laying the foundation for rescue centers to carry out optimal initial responses within the golden time.

According to the disclosure, even in a text-based satellite communication environment, through interworking between server-based conversational AI and a large language model (LLM), contextually customized emergency response guides can be provided to users or voice calls can be connected with emergency centers on behalf of the users. Accordingly, seamless bidirectional communication can be performed even in contexts where drivers have difficulty making direct calls, and the automatic translation and information search functions can significantly expand user experience (UX) to overseas travel and general convenience service areas.

### Brief Description of Drawings

The accompanying drawings, which are included as part of the detailed description to help understanding the disclosure, provide embodiments of the disclosure and, together with the detailed description, explain the technical idea of the disclosure.
FIG. 1 is a conceptual view of an embodiment of a non-terrestrial network system according to the disclosure.
FIG. 2 is a block diagram of a user terminal according to the disclosure.
FIG. 3 is a block diagram of an AI assistant server according to the disclosure.
FIG. 4 is an operational flowchart of a method of providing context awareness and context-specific service corresponding to the context awareness, performed in a satellite communication system according to the disclosure.
FIG. 5 is a conceptual view of data flow and interworking among components for each context type, performed in a satellite communication system according to the disclosure.
FIG. 6 is an operational flowchart of a method of generating and transmitting a command message, performed in a user terminal according to the disclosure.
FIG. 7 is a view of data flow for command message generation, summarization, and compression processes performed in a user terminal according to the disclosure.
FIG. 8 is an operational flowchart of an LLM linkage-based context analysis and response mode determination process performed in an AI assistant server according to the disclosure.
FIG. 9 is a view of an example of data processing and mode switching in which an AI assistant server according to the disclosure restructures LLM analysis results and provides the restructured analysis results to a user.
FIG. 10 is a view of an embodiment of data restructure, summarization, and compression performed in a user terminal and an AI assistant server according to the disclosure.
FIG. 11 is an operational flowchart in an emergency call mode performed by an AI assistant server according to the disclosure.
FIG. 12 is a view of an embodiment of server-based interactive TTS conversations performed in an emergency call mode according to the disclosure.
FIG. 13 is an operational flowchart in an emergency response guide mode performed by an AI assistant server according to the disclosure.
FIG. 14 is a view of an embodiment of on-device based interactive TTS conversations performed in an emergency response guide mode according to the disclosure.
FIG. 15 is an operational flowchart in a telephone reservation mode performed by an AI assistant server according to the disclosure.
FIG. 16 is a view of an embodiment of server-based interactive TTS conversations performed in a telephone reservation mode according to the disclosure.
FIG. 17 is an operational flowchart in an information search mode performed by an AI assistant server according to the disclosure.
FIG. 18 is a view of an embodiment of a server-based search method and a search result display method performed in an information search mode according to the disclosure.
FIG. 19 is a view of an embodiment of a minimum set of data (MSD) and a dataset that are selectively configured depending on a context type according to the disclosure.

### Mode for the Invention

It should be noted that the technical terms used herein are for the purpose of describing specific embodiments only and are not intended to limit the disclosure. The singular forms are intended to include the plural forms as well, unless the context clearly indicates otherwise. In general, a suffix such as "module" and "unit" may be used to refer to elements or components. Use of such a suffix herein is merely intended to facilitate description of the specification, and the suffix itself is not intended to give any special meaning or function.

In this specification, the terms "comprising" or "including" should not be construed to necessarily include all of the components or steps described in the specification, and some of the components or steps may not be included, or may include additional components or steps.

In describing the embodiments disclosed herein, moreover, the detailed description will be omitted when a specific description for publicly known technologies to which the disclosure pertains is judged to obscure the gist of the disclosure.

The accompanying drawings are used to help easily understand the technical idea of the disclosure and it should be understood that the idea of the disclosure is not limited by the accompanying drawings. The idea of the disclosure should be construed to extend to any alterations, equivalents, and substitutes besides the accompanying drawings. It should be understood that not only the embodiments described below but also combinations of embodiments may be included within the technical idea and scope of the disclosure as modifications, equivalents, or substitutes.

Hereinafter, embodiments disclosed in this specification will be described in detail with reference to the attached drawings.

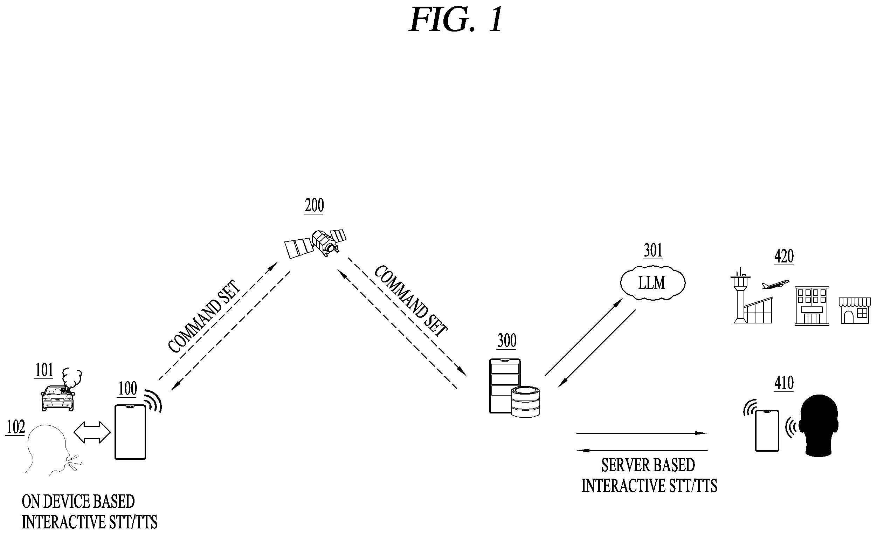

FIG. 1 is a conceptual view of an embodiment of a non-terrestrial network system according to the disclosure.

Referring to FIG. 1, a non-terrestrial network system according to the disclosure may include at least a user terminal 100, a satellite 200, and an AI assistant server 300. The non-terrestrial network system may further include external service providers, which interwork with the AI assistant server 300, for example, an emergency response center 410 and a telephone reservation center 420.

The user terminal 100 according to the disclosure may be configured to transmit an emergency message from a vehicle 101.

In this specification, the user terminal 100 is not limited to a portable device, such as a smartphone. It should be noted that in an embodiment, the vehicle 101 itself may perform the role of the user terminal 100 or components of the user terminal 100 may be implemented integrally in the vehicle 101. Therefore, in this specification, the user terminal 100 and the vehicle 101 may be used interchangeably or interpreted as the same entity depending on an implementation method, and the scope of the disclosure is not limited by a specific form.

The user terminal 100 may collect user voice data, vehicle sensor data, and UI input through the user terminal 100.

Here, vehicle information may be understood as a comprehensive concept that includes not only vehicle status information, such as vehicle speed, steering angle, acceleration, and collision sensor data, but also current vehicle location information, terrain information along a driving route, and communication environment information. Driver information may include a wide range of user information, including the number of occupants in a vehicle, user identification information for each occupant, user preferences, and past usage history, as well as driver status information, such as patient information and psychological state.

In an embodiment, the user terminal 100 may collect voice data of a user 102 (e.g., driver, occupants, etc.) located inside or outside the vehicle 101 and convert the voice data into data for transmission via satellite networks.

In this regard, the user terminal 100 may be configured to convert the voice data of the user 102 into text data and transmit and receive the converted text data to and from the satellite 200. To this end, the user terminal 100 may perform an interactive text-to-speech (TTS) function in an on-device manner. That is, the user terminal 100 itself may be configured to perform a function of converting user voice input into text data or converting a text-format response received from the AI assistant server 300 into a synthetic voice and outputting the converted results. This may be particularly useful in NTN environments where available radio resources are limited due to the nature of satellite communications, or where large-scale of voice data transmission are difficult during emergency contexts, such as emergency rescues.

In some embodiments, the user terminal 100 may, in some cases, directly transmit and receive voice data to and from the satellite 200. In this case, the user terminal 100 may be configured to compress a received voice signal using a predetermined voice codec or to convert the received voice signal into a voice frame in packet units and transmit the voice frame. The user terminal 100 may restore voice data received from the satellite 200 and output the restored voice data to the user.

In another embodiment, the user terminal 100 may collect sensor data from at least one sensor included in the vehicle 101. In this regard, sensor data may include data regarding vehicle status, vehicle surroundings, and driver status, which are acquired through driving sensors, biometric sensors, attitude recognition sensors, cameras, microphones, or collision detection sensors included in the vehicle 101.

In another embodiment, the user terminal 100 may receive not only the voice data of the user 102 but also input data generated by the user 102 operating the terminal 100. In this regard, input data may refer to user interface (UI) input generated by using button input, touch input, UI menu selection, or emergency call function.

The user terminal 100 may obtain at least one of vehicle information and driver information by analyzing at least one of voice data, sensor data, and user interface inputs (UI inputs) using an on-device artificial intelligence (AI) model.

The user terminal 100 may classify driver contexts by context types based on the acquired vehicle information and driver information.

Here, "context type" may be defined to represent the driver context. In an embodiment, the context type may be set to Type 0 to Type 4.

For example, 'Type 0' may indicate an 'emergency call' context as a context that requires immediate rescue (e.g. direct 911 call). Type 1 and Type 2 may indicate 'emergency response guide' contexts where life is not in danger but an emergency response manual is needed for medical advices or simple measures. Type 3 or Type 4 may indicate a 'telephone reservation' or 'information search' context, as a telephone reservation and information search for general context-based information conversation or guidance service.

In some embodiments, the classification system of context types in this specification is not limited to Type 0 to Type 4 described above. In embodiments, new context types may be further defined as types of multimodal data collected expand or service scenarios provided through the system according to the disclosure diversify. It should be noted that a plurality of preset context types may be operated as a single integrated category depending on a method of operating the system. Therefore, Type 0 to Type 4 exemplified in this specification are merely functional distinctions to help understanding of the invention, and the scope of the disclosure is not limited by specific names or numbers.

The context type may be determined based on a trigger type that indicates an input type triggered by any one of user voice input, sensor input, and UI manipulation input.

For example, in case that a vehicle collision sensor is detected or the pitch and intensity of the user's voice are analyzed to be at least a preset threshold, the context type may be determined as 'Type 0 (emergency call),' which has the highest priority. In another example, in case that the analysis of location information included in input information reveals that the vehicle is located in a high-risk area for secondary accidents, such as a highway or tunnel, or in a communication dead zone, the user terminal 100 may upgrade an emergency urgency level by applying a preset weight and determine the context type accordingly.

The user terminal 100 may generate a corresponding command message based on vehicle information and driver information. For example, in case that vehicle information and driver information indicate an emergency context, such as sudden braking, collision risk, loss of consciousness, or abnormal vital signs, the user terminal 100 may generate a command message corresponding to a rescue request.

The user terminal 100 may be configured to extract necessary data corresponding to the classified context type. In an embodiment, in case that the context type is an emergency call or emergency response guide, the necessary data may include at least one of whether a crash has occurred, vehicle identification information, location information, vehicle speed, and occupant information. In another embodiment, in case that the context type is a telephone reservation or information search, the necessary data may include at least one of location information, user information, vehicle information, and service request information.

The user terminal 100 may generate a command message including text data corresponding to the context type, the extracted necessary data, and the voice data, summarize and compress the command message, and transmit the command message to the satellite 200.

At this time, the user terminal 100 may determine a summarization level or compression ratio differently depending on the context type and satellite communication link quality. For example, in an emergency context or low communication quality context, the text summarization level may be increased or a high compression algorithm may be applied to improve a data transmission success rate.

The AI assistant server 300 may transmit and receive voice data or text data to and from the satellite 200, and provide a service that corresponds to the user context by interworking with the emergency response center 410 and the telephone reservation center 420.

The AI assistant server 300 according to the disclosure may operate by interworking with a gateway infrastructure which is operated by a satellite communication operator. For example, the AI assistant server 300 may operate at a location adjacent to a gateway device, which connects the satellite 200 to terrestrial networks, or may be configured as a server cluster inside the gateway device. In another embodiment, the AI assistant server 300 may be arranged in an external cloud environment established on a public network or a private network to receive and process data transmitted through a satellite gateway.

The AI assistant server 300 may be configured to operate by interworking with a large language model (LLM) 310. Here, the LLM 301 may refer to an artificial intelligence (AI) model that performs natural language understanding and response generation for user input by pre-training a large-scale corpus. The LLM 301 may be provided as a cloud AI service.

The AI assistant server 300 according to the disclosure may be configured to analyze a command message received from the user terminal 100 and perform an operation corresponding to a context type included in the message.

In an embodiment, when receiving a command message with a context type of Type 0, the AI assistant server 300 may be configured to activate a server-based conversational AI to interwork with the emergency response center 410, thereby transmitting an emergency rescue request and perform a voice call to the emergency response center 410 on behalf of the user.

In another embodiment, when receiving a command message with a context type of Type 1 or Type 2, the AI assistant server 300 may generate a prompt appropriate for the context and provide the prompt to the LLM 301. In response to this, the server 300 may receive an LLM response including context analysis results from the LLM 301 and reanalyze the received LLM response. At this time, in case that the context is judged to be serious, the server 300 may automatically switch to an emergency call mode and perform a fail-safe logic for connection to the emergency response center 410. Otherwise, the server 300 may restructure the LLM response and transmit the restructured LLM response to the user terminal 100.

In another embodiment, when receiving a command message with a context type of Type 3 or Type 4, the AI assistant server 300 may provide a corresponding service by interworking with the telephone reservation center 420 or an external search engine. To this end, the AI assistant server 300 may perform automatic reservation or information search, and generate a response message including a result of the automatic reservation or information search.

Thereafter, the user terminal 100 may receive a response message through the satellite 200, and translate text data included in the response message into a target language or convert the text data into voice through TTS to provide guidance to the user, thereby realizing bidirectional communication.

In some embodiments, the classification of context types processed by the AI assistant server 300 in this specification and the corresponding service modes are not limited to Type 0 to Type 4 described above. The server 300 according to the disclosure may additionally define new context types and dynamically expand new service modes corresponding thereto as types of multimodal data collected from the user terminal 100 expand or service scenarios provided through the system according to the disclosure diversify. A plurality of preset context types and service modes may be operated by being merged into a single integrated intelligent service category according to load balancing or operational policies of the system, or conversely, a specific type may be diversified and operated according to detailed conditions. Therefore, it should be noted that each type Type 0 to Type 4 and its corresponding service functions exemplified in this specification are merely illustrative to help understanding of the invention, and the technical scope of the disclosure is not limited by specific names, numbers, or fixed mapping relationships.

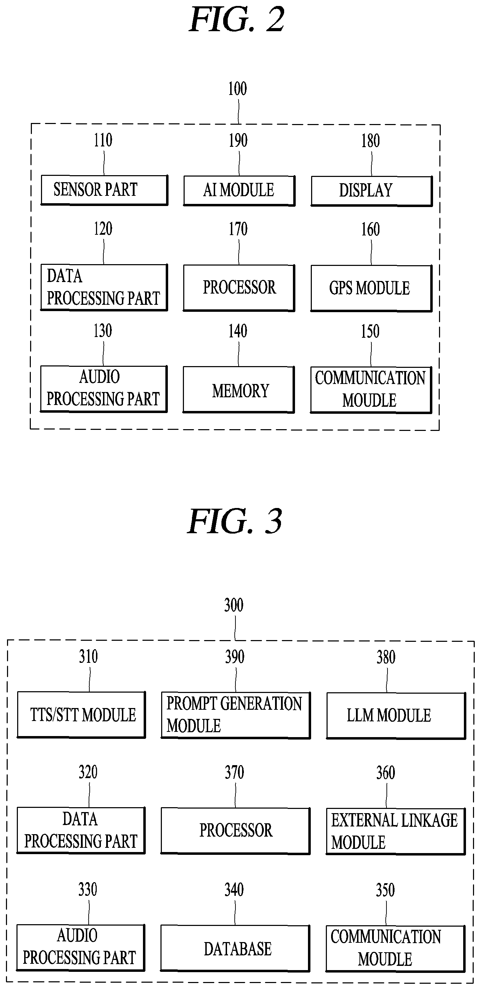

FIG. 2 is a block diagram of a user terminal 100 according to the disclosure.

Referring to FIG. 2, the user terminal 100 according to the disclosure may be configured to include at least a sensor part 110, a data processing part 120, an audio processing part 130, a memory 140, a communication module 150, a GPS module 160, a processor 170, a display 180, and an AI module 190. Each of the components may transmit and receive data to and from each other through buses or other electrical connection members and may be operably connected under the control of the processor 170.

The sensor part 110 may be an integrated detection unit for detecting changes in physical statuses of the vehicle and driver, and may perform motion detection, vehicle status, and bio-signal detection functions depending on detection purpose.

In an embodiment, the sensor part 110 may include an inertial measurement unit (IMU) to detect in real time whether the vehicle is suddenly decelerated, impacted, or overturned. The sensor part 110 may collect airbag deployment signals and tire pressure information, through interworking with an internal network of the vehicle, to cross-verify whether an accident has occurred. The sensor part 110 may measure the driver's vital signals to determine whether the driver is conscious and an emergency urgency level. Data collected from each sensor may be transmitted to the data processing part 120 and, after undergoing synchronization and noise filtering processes, may be used as input data for context determination of the AI module 190.

The data processing part 120 may refer to a preprocessing module which processes data (raw data) collected from the sensor part 110 into a form suitable to be processed by the AI module 190.

In an embodiment, the data processing part 120 may apply a low-pass filter or Kalman filter algorithm to remove noise generated during driving and extract only meaningful impact signals. The data processing part 120 may ensure data consistency by correcting or interpolating a sampling rate based on timestamps of heterogeneous sensor data collected at different cycles. The data processing part 120 may normalize the collected data and convert the data into a vector or tensor form that meets the input specifications of the AI module 190, thereby optimizing computational efficiency. The preprocessed data may be transmitted to the AI module 190 and used as key input data for determining accident type classification and emergency urgency level.

The audio processing part 130 may perform the role of converting an analog voice signal input through a microphone into a digital signal. To this end, the audio processing part 130 may be configured to include a digital signal processor (DSP) which extracts voice data and acoustic features through a preset algorithm.

In an embodiment, the audio processing part 130 may perform a function of converting a voice signal into text data using an STT model stored in the memory 140. At the same time, the audio processing part 130 may analyze input voice waveforms in a frequency domain or time domain to extract acoustic feature vectors, which include pitch, intensity, frequency variation, amplitude variation, and speech rate. The audio processing part 130 may be configured to transmit the extracted feature vectors to the AI module 190.

The audio processing part 130 may include a function of converting text data into a synthetic voice signal by utilizing a TTS model stored in the memory 140. Specifically, the audio processing part 130 may convert a text format response message received from the communication module 150 or AI module 190 into a synthetic voice signal through the TTS model. Afterwards, the audio processing part 130 may control the converted signal to be output to a speaker through a digital-to-analog converter (DAC).

The memory 140 may refer to a storage medium which stores data, programs, and algorithms necessary for the operation of the terminal. In an embodiment, the memory 140 may be configured to include at least one of various types of non-volatile or volatile memories, such as flash memory, RAM, and ROM.

The memory 140 may perform a function of temporarily or permanently storing programs or applications for controlling the processor 170, or input/output data. In an embodiment, the memory 140 may store offline map data, which includes road networks and terrain information, to prepare for contexts where network connection is unavailable. This may allow the terminal to identify geographical environment information even in communication dead zones.

The memory 140 may store a pre-learned artificial intelligence model and weight parameters required for driving the AI module 190 to be described later. Through this, the memory 140 can provide an environment in which the terminal can independently perform on-device operations without communication with an external server.

The GPS module 160 may refer to a positioning device which receives signals from at least one global navigation satellite system (GNSS), and calculates current location coordinates (latitude, longitude, altitude, and the like) and movement speed of the terminal in real time. In an embodiment, the GPS module 160 may be configured to comprehensively support various global satellite navigation systems, including GPS, GLONASS, Galileo, and BeiDou.

The GPS module 160 may include a function of generating uninterrupted location information by performing dead reckoning through interworking with IMU data of the sensor part 110, to ensure precise positioning in dead zones where satellite signal reception is difficult, such as tunnels or underground parking lots.

The GPS module 160 may operate independently regardless of whether a terrestrial network communication connection is established, and in particular, in the event of a satellite communication-based emergency context, the GPS module 160 may obtain precise coordinate information on an accident site and transmit the coordinate information to the server 300 or emergency response center 410.

In some embodiments, the GPS module 160 and the memory 140 may work together to perform location-based context awareness of the terminal. In an embodiment, the processor 170 may match the current coordinates of the terminal tracked in real time through the GPS module 160 with offline map data stored in the memory 140. Through this, the processor 170 can determine a specific geographical environment, such as whether the current vehicle is on a highway or in a mountainous area. The location information may be used as an important basis for determining an emergency urgency level during a context awareness process to be described later.

The AI module 190 may refer to an on-device artificial intelligence (AI) processing device which is activated by loading an AI model stored in the memory 140 and comprehensively judges a context based on collected multimodal data. The AI module 190 may be implemented as a separate neural network processing unit (NPU) or may be installed as a type of a software algorithm inside the processor 170. The AI module 190 may use lightweight models to enable independent inference even when network connectivity is interrupted.

In an embodiment, the AI module 190 may perform multimodal context analysis. Specifically, the AI module 190 may integrate and analyze text data from the audio processing part 130, impact data from the sensor part 110, and geographical environment information. Through this, the AI module 190 may classify in detail whether the corresponding context is a 'simple contact accident' or a 'life-threatening rollover accident' and calculate an emergency urgency level accordingly.

The AI module 190 may perform a driver psychological state analysis function. To this end, the AI module 190 may infer a psychological state by analyzing acoustic features, such as pitch, tremor, and speech rate of voice waveforms. For example, the AI module 190 may identify severe voice tremors as a state of 'panic' and unclear speech as 'reduced consciousness.' This psychological state information may be included as metadata in a rescue request message.

Furthermore, the AI module 190 may perform text optimization suitable for a satellite communication environment. The AI module 190 may reconstruct a sentence by removing unnecessary particles or redundant expressions from text and extracting only core keywords. Through this intelligent compression process, the AI module 190 can be configured to minimize the size of data packets and increase a transmission success rate.

The communication module 150 may refer to a hybrid communication interface which supports both terrestrial network communication and non-terrestrial network communication. Specifically, the communication module 150 may be configured to include a first communication modem and a second communication modem which are physically or logically divided.

In an embodiment, the first communication modem may refer to a broadband mobile communication modem which performs communication with a terrestrial base station, such as LTE or 5G new radio (NR). The first communication modem may be used for regular vehicle infotainment or general data communication. The second communication modem may refer to a satellite-dedicated modem (e.g., NR-NTN or IoT-NTN modem) which performs direct communication with low earth orbit (LEO) or geostationary earth orbit (GEO) satellites.

The processor 170 may control the communication module 150 to select a modem appropriate for a context. For example, in an urban area where terrestrial reference signal received power (RSRP) is equal to or greater than a threshold value, the first communication modem may be activated to maintain high-speed communication. On the other hand, an accident site may be determined to be a terrestrial network shadow area, such as a remote mountainous area or at sea. In another example, an antenna of the first communication modem may be damaged due to an impact of the accident, making connection impossible. In this case, the processor 170 may immediately wake up the second communication modem to form a satellite link. Such dual modem-based automatic switching may be configured to ensure a reliable external transmission path for rescue signals even in poor communication environments.

The display 180 may refer to an input/output device which provides visual information to a user and receives touch input. In terms of hardware, the display 180 may perform the role of generating a driving signal by converting a video signal, a data signal, and an on screen display (OSD) signal, into R, G, and B signals, respectively.

In an embodiment, the display 180 may visually output system status information under the control of the processor 170. Specifically, the display 180 may display a transmission progress status of a command message and a satellite connection strength.

The display 180 may be configured to render a graphical user interface (GUI) corresponding to a context type identified by the processor 170. For example, the display 180 may display a map based on location information of the GPS module 160 and map data of the memory 140. In another example, the display 180 may output emergency response guide images and text data.

The display 180 may support a function of converting and outputting a received response message into the user's native language under the control of the processor 170.

The processor 170 may refer to a central processing unit (main controller) which controls the overall operation of the user terminal 100 and performs operations. The processor 170 may be configured to determine an emergency context by analyzing data collected from each module and to control the generation of a command message. In terms of hardware, the processor 170 may be implemented as an application processor (AP), a central processing unit (CPU), or a microcontroller (MCU).

The processor 170 may perform integrated logic which controls signal flow between components. Specifically, when a physical impact signal exceeding a threshold value is input from the sensor part 110, the processor 170 may recognize the signal as an interrupt signal. Accordingly, the processor 170 can control the GPS module 160 and the AI module 190, which were in a standby mode, to apply a wake-up signal.

The processor 170 may receive an emergency urgency level determination result and a terrestrial network connection failure signal output from the AI module 190. Based on this, the processor 170 may control the communication module 150 to instruct the communication model 150 to immediately switch the communication path to a satellite modem.

Furthermore, the processor 170 may identify a context type by analyzing a header of the received response message. The processor 170 may then generate video and audio data signals corresponding to the identified type and integrally control the generated data signals to be transmitted to the display 180 and audio processing part 130, respectively.

FIG. 3 is a block diagram of an AI assistant server 300 according to the disclosure. Referring to FIG. 3, the AI assistant server 300 according to the disclosure may be configured to include at least a TTS/STT module 310, a data processing part 320, an audio processing part 330, a database 340, a communication module 350, an external linkage module 360, a processor 370, an LLM module 380, and a prompt generation module 390. Each of the components may transmit and receive data to and from each other through buses or other electrical connection members and may be operably connected under the control of the processor 370.

The TTS/STT module 310 may refer to an engine which performs server-based high-performance speech-to-text conversion and dialogue processing functions based on signals received from the audio processing part 330.

In an embodiment, the TTS/STT module 310 may convert compressed voice data transmitted from the terminal 100 into high-precision text data through a speech-to-text (STT) function. In particular, the TTS/STT module310 may utilize large-capacity computational resources of a server, to secure a high recognition rate even in emergency contexts where voices are mixed with noise or speech is unclear.

The TTS/STT module 310 may have an interactive text-to-speech (TTS) function built therein. This can allow for the synthesis of voices, to which context-appropriate intonation and emotion are reflected, beyond simple text recitation. For example, the TTS/STT module 310 may generate a voice response in a calm and clear tone which can reassure a driver in an emergency call mode, while generating a voice response in a natural conversational tone in a telephone reservation mode.

Furthermore, the TTS/STT module 310 may support multilingual interpretation and translation functions. This can help bridge the language barrier by performing real-time voice conversion between the user's native language and a local language when an accident occurs or a reservation is made overseas.

The data processing part 320 may refer to a preprocessing module which processes a compressed command message received through the communication module 350. Specifically, the data processing part 320 may perform a decompression function to restore a received compressed packet to its original data form.

Afterwards, the data processing part 320 may precisely analyze header field and payload of the command message. Through this, the data processing part 320 can separate multimodal data, such as text, sensor values, and audio data, and secure time-series consistency of each data. The data processing part 320 may perform the role of normalizing separated data into a standard format (e.g., JSON or vector) which can be processed by a processor 375 or LLM of the server 300.

The data processing part 320 may perform a variable compression function for a response message to be transmitted to the terminal 100. The data processing part 320 may dynamically determine a compression ratio based on current satellite communication link quality or an urgency level of an identified context. For example, when communication conditions are unstable or an urgency level is high, the data processing part 320 may apply a text-oriented high-compression algorithm. This can minimize the data size and reduce transmission delay.

The audio processing part 330 may refer to a signal processing device that sets up and manages voice conversation environments of the server 300. Specifically, the audio processing part 330 may perform the role of controlling acoustic signals generated in a call line with an external organization (e.g., emergency centers or restaurants).

In an embodiment, the audio processing part 330 may apply high-performance acoustic echo cancellation (AEC) and noise reduction algorithms to a received voice signal. Through this, the audio processing part 330 can refine the voice signal while maintaining the purity of the voice signal even in a noisy environment. The audio processing part 330 may transmit the refined voice signal to the TTS/STT module 310. This may serve as an essential preprocessing step to maximize a voice recognition rate of the STT engine.

The audio processing part 330 may serve as a gateway which converts synthetic voice data generated by the TTS/STT module 310 into a codec suitable for an external public switched telephone network (PSTN) or VoIP protocol and transmits the converted data.

The database 340 may refer to a large-capacity storage medium which stores data and algorithms required for the operation of the server 300. In an embodiment, the database 340 may store essential setting data for interworking with the external LLM 301. Specifically, the database 340 may store a prompt template for each context type used by the prompt generation module 390.

The database 340 may store API authentication keys and connection information for external service linkage. Through this, the database 340 can be configured to support environments in which the server 300 can seamlessly interwork with the LLM 301 to provide intelligent services based on natural language processing.

The communication module 350 may refer to a hybrid communication interface which supports both terrestrial network communication and non-terrestrial network communication. In some embodiments, the communication module 350 may include a satellite network gateway interface for communication with the user terminal 100. The communication module 350 may be configured to include an external public network interface for interfacing with external services.

In an embodiment, the communication module 350 may receive a command message transmitted from the user terminal 100 via a satellite. The communication module 350 may perform the role of transmitting a response message processed within the server back through a satellite network.

Furthermore, the communication module 350 may establish a communication channel with the emergency response center 410 and a general service provider (e.g., the telephone reservation center 420) via an external public network. Through this, the communication module 350 can perform a function of immediately transmitting accident context information to an emergency center or transmitting and receiving restaurant and accommodation reservation data to and from a service provider server.

At this time, transmission and reception of messages between satellites may be performed in compliance with standard non-terrestrial network (NTN) specifications, such as NR-NTN and IoT-NTN.

The external linkage module 360 may serve as a gateway which transmits processing results of the server 300 to an external service and integrates resulting data.

In an embodiment, the external linkage module 360 may determine a target to be connected based on an identified context type. For example, in an emergency context, the external linkage module 360 may establish an immediate call connection with the emergency response center 410 or transmit accident data to the emergency response center 410. In case of an information search or reservation context, the external linkage module 360 may transmit an API query to an external search engine 430 or a server of the general service provider (e.g., the telephone reservation center 420). The external linkage module 360 may collect and refine search results or reservation confirmation information received from outside. Through this, the server 300 can be configured to integrate data into a processable form and transmit the integrated data to the processor 375.

The LLM module 380 may refer to a computational device which performs unstructured data analysis through interworking with the external LLM 301 providing a cloud AI service. In an embodiment, the LLM module 380 may serve as an interface which transmits a query received from the prompt generation module 390 to the external LLM 301. Thereafter, the LLM module 380 may transmit analysis results received from the external LLM 301 to the processor 370.

The prompt generation module 390 may refer to a software engine which analyzes standardized terminal information received from the data processing part 320 and dynamically generates 'context-aware prompt' based on a natural language, so that the LLM module 380 can perform optimal inference. In some embodiments, the prompt generation module 390 may restructure heterogeneous multimodal data, such as text, sensor values, and bio-signals transmitted from the terminal, into a single integrated context which can be understood by the LLM.

In an embodiment, the prompt generation module 390 may specify a system command by utilizing a context-specific template stored in the database 340. For example, when rollover accident data is detected, the prompt generation module 390 may control response quality of the LLM by assigning the role of "Act as a skilled emergency responder from now on" to the LLM. The prompt generation module 390 may adjust urgency level of the LLM request and response style based on a geographic location (e.g., communication dead zone) and the driver's psychological state (e.g., fear or unconsciousness). Accordingly, the prompt generation module 390 can be configured to provide concise and clear guidance in emergencies and induce detailed and friendly answers in general reservations.

The processor 370 may refer to a central processing unit (main controller) which controls the overall operation of the server 300 and performs operations for large-scale data processing. In some embodiments, the processor 370 may perform a function of identifying a type of context which the terminal is currently in by analyzing header information or payload of a received command message.

The processor 370 may perform integrated control logic which determines the operation of a system according to the identified context type. The processor 370 may selectively activate the LLM module 380, the TTS/STT module 310, and the external linkage module 360 according to the context. Through this, the processor 370 can be configured to provide services by branching into processing logic optimized for each operation mode, such as emergency call, information search, and reservation.

In some embodiments, the user terminal 100 and AI assistant server 300 shown in FIGS. 1 to 3 are merely an example of the disclosure, and therefore some of those shown components may be integrated, added, or omitted depending on the specifications of the actually implemented user terminal 100 and AI assistant server 300.

For example, two or more components may be integrated into one component, or one component may be divided into two or more components, as needed. Functions performed in each block are intended to explain an embodiment of the disclosure, and specific operations or devices thereof do not limit the scope of the disclosure.

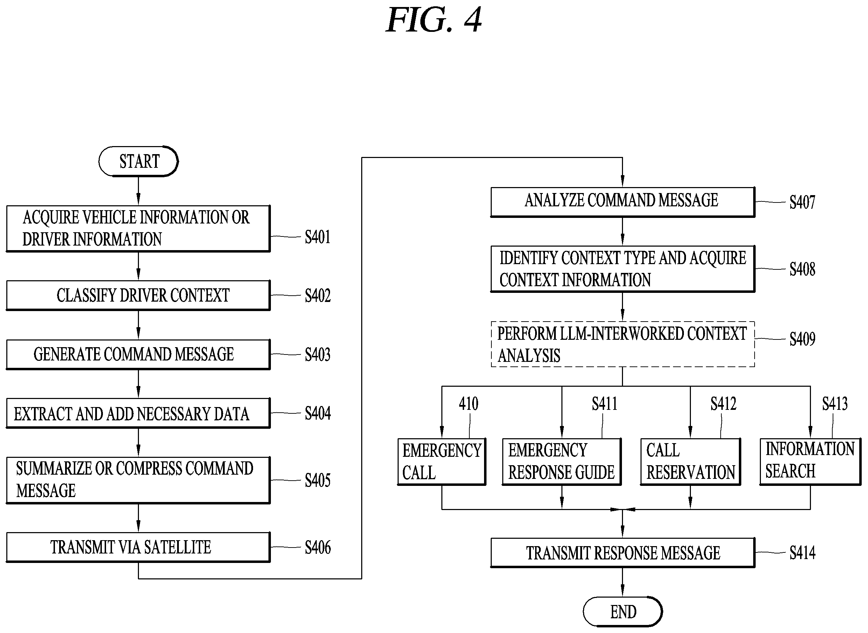

Hereinafter, descriptions will be given of the command message configuration and transmission operation based on vehicle and driver statuses according to the disclosure and corresponding response operations for each context. In this regard, FIG. 4 is an operational flowchart of a method of providing context awareness and context-specific service corresponding to the context awareness, performed in a satellite communication system according to the disclosure.

Referring to FIGS. 1 to 4, a method of providing a service based on a driver context performed in a satellite communication system may be divided into a process of determining, by the user terminal 100, a driver context and generating and transmitting a related command message (S401 to S406), and a process of receiving and processing, by the AI assistant server 300, the command message (S407 to S414).

First, the user terminal 100 may acquire at least one of vehicle information and driver information based on at least one of collected voice data, sensor data, and user input data (S401).

In this regard, the user terminal 100 may collect data through sensors, microphones, cameras, or user interfaces (UIs) inside and outside the vehicle. Specifically, the terminal 100 may monitor and receive in real time sensor data obtained by detecting whether a vehicle has collided, voice data including the driver's speech, and the user's touch input.

The user terminal 100 may classify a driver context into at least one of a plurality of context types based on the acquired information (S402).

In this regard, the user terminal 100 may determine whether an emergency context exists or an emergency urgency level based on the acquired information. The context type may be classified based on whether the emergency context exists or the emergency urgency level.

In an embodiment, the user terminal 100 may determine whether an emergency context exists or an emergency urgency level by analyzing the vehicle information and driver information using an on-device AI model. At this time, the context type may be classified based on whether the emergency context exists or the emergency urgency level, and may include at least one of an emergency call, an emergency response guide, a telephone reservation, and an information search. For example, when a result of the determination is the emergency context, the user terminal 100 may classify the driver context as a context requiring an emergency call or emergency response guide. On the other hand, when a result of the determination is not an emergency context, the terminal 100 may classify the driver context as a general assistant service request context, such as a telephone reservation or information search.

In some embodiments, the user terminal 100 may determine the emergency urgency level by reflecting the driver's psychological state and positional characteristics.

In an embodiment, the user terminal 100 may obtain the driver's psychology index by analyzing acoustic features, including at least one of pitch, intensity, and speech rate of voice data. Here, the driver's psychology index may mean a quantitative value or grade that indexes the driver's psychological state, such as panic, fear, and calmness. The terminal 100 may determine the emergency urgency level by reflecting the calculated driver's psychology index.

In another embodiment, the user terminal 100 may obtain location information from sensor data and calculate a risk weight corresponding to the obtained location information. Here, sensor data may be understood as a concept that includes not only location data acquired by the GPS module 160, but also location and terrain information estimated based on inertial sensor data of the sensor part 110.

In this regard, the user terminal 100 may obtain the current vehicle location information by analyzing the collected sensor data and calculate a risk weight corresponding to topographical or communicative environmental characteristics of the location. For example, when the vehicle 101 is located in a highway, tunnel, or a remote area with a communication dead zone, the higher risk may be assigned because the accident risk or the difficulty of rescue is high. The user terminal 100 may determine the emergency urgency level by reflecting the calculated risk weight.

The user terminal 100 may classify the driver context as 'Type 0 (emergency call)' when the calculated emergency urgency level is higher than or equal to a first threshold value, thereby controlling an immediate connection with the emergency response center 410. On the other hand, when the emergency urgency level is between the first threshold value and a second threshold value, the user terminal 100 may classify the driver context as 'Type 1 (emergency response guide)' by determining that an immediate emergency call is not necessary but medical advices are needed. In this case, the user terminal 100 may receive an emergency response guide optimized for the driver context and provide the emergency response guide to the user 102.

The user terminal 100 may generate a command message based on the context type and acquired information (S403).

In this regard, the user terminal 100 may convert voice data into text data by using a speech-to-text (STT) model and add the converted text data to the command message.

In an embodiment, the user terminal 100 may perform signal correction by removing background noise from the voice data before converting the voice data into text. This is to suppress a voice recognition rate from deteriorating due to driving wind noise or accident impact noise, and to obtain a clear voice signal.

The user terminal 100 may perform cross-validation with sensor data to ensure the reliability of the converted text data. In an embodiment, the user terminal 100 may determine whether the text data is different from collected sensor data. When the text data and the sensor data are different, the user terminal 100 may perform content correction on the text data by applying priority to the sensor data.

For example, when the driver utters "I'm okay" due to shock but the sensor data indicates 'airbag deployment' and 'vehicle rollover,' the user terminal 100 may prioritize the sensor data. In this case, the user terminal 100 may generate a context message by forcibly inserting a phrase "[Automatic detection: Overturning accident occurred]" into text data or correcting the text data.

In an embodiment, the generated command message may have a structure including a header field in which the identified context type is recorded, and a payload field in which the converted or corrected text data is recorded.

The user terminal 100 may extract necessary data associated with the context type and add the necessary data to the generated command message (S404).

In this regard, the user terminal 100 may selectively extract necessary data that matches the determined context type from the memory or a vehicle network (CAN).

In an embodiment, when the context type is an emergency call or emergency response guide, the required data may include at least one of whether a crash has occurred, vehicle identification information, location information, vehicle speed, and occupant information. For example, the necessary data may include collision sensor values, vehicle identification number (VIN), GPS coordinates, speed at the time of an accident, and number of occupants, and may configure a minimum set of data (MSD) essential for rescue operations.

On the other hand, when the context type is a telephone reservation or information search, the necessary data may include at least one of location information, user information, vehicle information, and service request information. For example, the necessary data may include a current vehicle location, the name of a person making a reservation, a vehicle type, and a search keyword entered by the user (e.g., 'nearby restaurants') or reservation time information.

The user terminal 100 may configure a transmission message by adding the necessary data extracted according to the context to the payload of the command message.

The user terminal 100 may perform summarization or compression on the command message (S405).

In this regard, to increase data transmission efficiency in consideration of a limited satellite bandwidth, the user terminal 100 may perform data lightweighting to reduce the size of the command message. Here, data lightweighting may mean summarizing or compressing the command message. At this time, the summarization level or compression rate for the command message may be applied differently depending on at least one of the context type and satellite communication link quality. That is, the terminal 100 may perform variable compression policies depending on the urgency of the context or the communication status.

In an embodiment, when the context type is an emergency call or an emergency response guide or when the satellite communication link quality is below a threshold, the user terminal 100 may compress the command message at a first compression ratio. Here, the first compression ratio may be a lossless compression method that ensures information accuracy by minimizing data loss, or a high compression method that extremely reduces a packet size by prioritizing a transmission success rate.

On the other hand, when the context type is a telephone reservation or information search and the satellite communication link quality is above the threshold, the user terminal 100 may compress the command message at a second compression ratio lower than the first compression ratio. This is to preserve data quality and more details in good communication environments and non-urgent contexts.

The user terminal 100 may apply different summarization levels depending on contexts. For example, in an emergency context, the user terminal 100 may perform 'high-intensity summarization' that extracts only core keywords, such as "crash detected or unconscious." In another example, in non-emergency contexts, the user terminal 100 may perform 'low-intensity summarization' that maintains the context of a sentence, such as "I would like to make a reservation at an Italian restaurant near the Gangnam station."

The user terminal 100 may transmit the lightweighted command message, i.e., a summarized or compressed command message, through the satellite (S406).

In some embodiments, the AI assistant server 300 that has received the command message via the satellite 200 may analyze the received command message (S407). To this end, the server 300 may first decompress the received command message.

Next, the server 300 may analyze the command message to identify the context type included in the command message and acquire context information (S408).

In this regard, the server 300 may parse the header information of the restored (decompressed) command message to identify the context type indicating the type of service requested by the terminal 100. Afterwards, the server 300 may analyze the command message and acquire the context information. In this regard, the server 300 may acquire at least one of rescue request information, emergency response information, reservation information, and search information depending on the identified context type.

In an embodiment, the rescue request information may include latitude, longitude, crash point, and airbags deployment status of the vehicle. The emergency response information may include a patient's state of consciousness, location of pain, and whether the patient is breathing. The reservation information may include the name of person making a reservation, number of people, desired time, and type of location. The search information may include search keywords, search radius, and category information.

The server 300 may operate in at least one mode among an emergency call mode, an emergency response guide mode, a telephone reservation mode, and an information search mode depending on the identified context type. For example, the server 300 may operate by branching into at least one mode among the following steps (S410 to S413) based on the identified context type.

In an embodiment, when the identified context type is an emergency call, the AI assistant server 300 may operate in an emergency call mode (S410).

In this regard, the server 300 may wake up server-based conversational AI to connect an emergency call with the emergency response center 410.

The server 300 may perform a voice-based conversation with the connected emergency response center 410 based on the acquired rescue request information. In some embodiments, the server 300 may process real-time conversations by converting received text data into voice (TTS) or converting the voice of a responder into text (STT).

Thereafter, the server 300 may generate a response message to be transmitted to the user terminal 100, where the response message includes feedback information including dispatch information and action results of an emergency responder.

In some embodiments, when the identified context type is an emergency response guide, the server 300 may further perform context analysis (S409) through interworking with the LLM 301, prior to the emergency response guide step (S411).

In this regard, the context analysis (S409) may include the following steps. First, the server 300 may construct an LLM request requesting context analysis using a context-specific prompt model and transmit the constructed LLM request to the LLM 301.

In response to the LLM request transmission, the server 300 may receive an LLM response including context analysis results. The server 300 may analyze and reconstruct (restructure) the received LLM response.

When it is determined that the analysis result of the LLM is above a preset urgency level based on a result of reconstructing the LLM response, the server 300 may automatically switch to the emergency call mode (S410) and connect an emergency call with the emergency response center.

The server 300 may continue to operate in the emergency response guide (S411), analyze the reconstructed LLM response, acquire guide texts for a plurality of steps, and generate a response message including the acquired guide texts for the plurality of steps.

In another embodiment, when the identified context type is a telephone reservation, the AI assistant server 300 may operate in the telephone reservation mode (S412).

In this regard, the server 300 may automatically perform a reservation procedure based on the acquired reservation information. First, the server 300 may wake up the server-based conversational AI to connect a call with a reservation target (e.g., the telephone reservation center 420 of FIG. 1) included in the reservation information.

The server 300 may perform voice-based conversations with the reservation target based on the reservation information. This may mean a process in which the server 300 uses TTS/STT technology to communicate with the other party, such as a restaurant employee, and confirms a reservation on behalf of the user.

Finally, the server 300 may generate a response message to include feedback information including whether the reservation was successful and reservation details. This response message may be transmitted to the user terminal 100 and used to inform the user of the reservation confirmation details.

In another embodiment, when the identified context type is information search, the AI assistant server 300 may operate in the information search mode (S413).

In this regard, the steps operating in the information search mode may be performed as follows. First, the server 300 may generate a search query based on the acquired search information. The server 300 may perform information search through interworking with an external search engine.

The server 300 may extract key information that matches the user's intent from result data of the information search and perform text summarization based on the extracted key information. For example, the server 300 may lightweight data by summarizing only essential information, such as addresses or contact information, for the top two or three best locations, instead of dozens of search result links.

Finally, the server 300 may generate a response message to include the summarized text data. The response message may be transmitted to the terminal 100 via the satellite 200 to be provided to the user.

In some embodiments, the AI assistant server 300 may transmit the generated response message, including the performance results of each step (S410 to S413) to the user terminal 100 (S414). In an embodiment, the server 300 may summarize and compress the generated response message and transmit the resulting message to the user terminal 100 via the satellite 200. At this time, the response message may include a control command, which causes the user terminal 100 to provide voice guidance through an on-device TTS or output information automatically translated according to a country code.

Accordingly, the user terminal 100 can receive the response message to the transmitted command message from the server 300 via the satellite 200. The user terminal 100 may perform a response action associated with the received response message. In an embodiment, the user terminal 100 may perform an action to provide voice guidance on emergency response guide information according to the TTS command included in the response message, or to display reservation confirmation details or summarization results of the information search on a UI screen.

In some embodiments, in the above description, steps S407 to S414 are described as being performed by the AI assistant server 300, but the technical idea of the disclosure is not limited thereto. According to various embodiments of the disclosure, at least some of those steps S407 to S414 may be independently performed by a processor and an on-device AI model mounted on the user terminal 100, in some cases. It should be noted that the above processes may be organically distributed and processed among the user terminal 100, satellite 200, and server 300 depending on system environments.

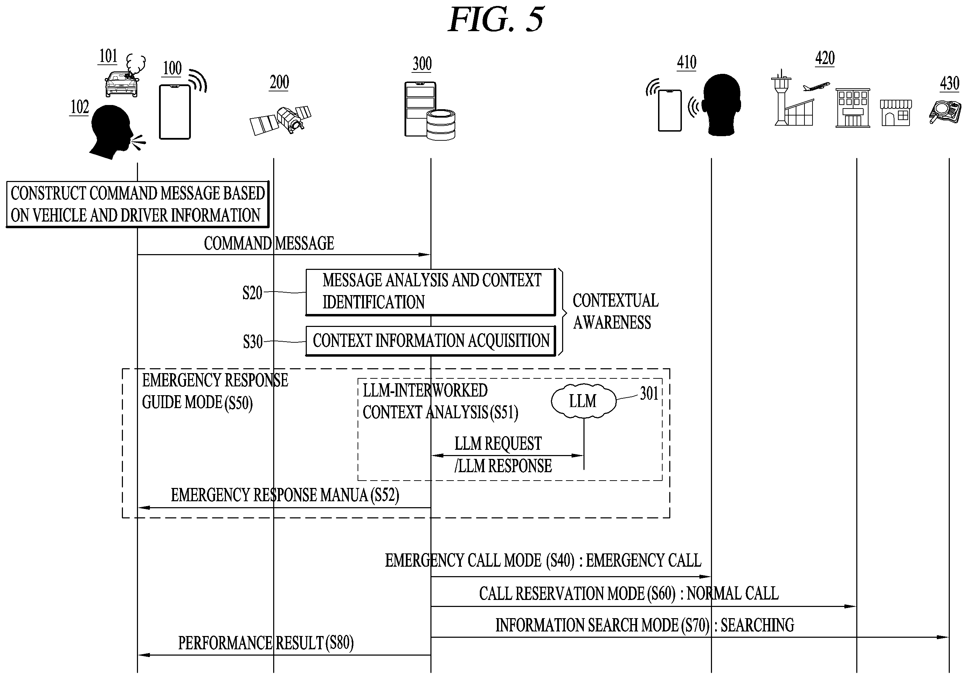

Hereinafter, the data flow and interworking method among components for each context type, performed in a satellite communication system, according to the disclosure, will be described. In this regard, FIG. 5 is a conceptual view of data flow and interworking among components for each context type, performed in a satellite communication system according to the disclosure.

Referring to FIGS. 1 to 5, a method of providing a service based on a driver context performed in a satellite communication system may be implemented through interworking among the user terminal 100, the satellite 200, the AI assistant server 300, and external service entities. In an embodiment, the external service entities may include the LLM 301, the emergency response center 410, the general service provider 420, such as telephone reservation, and an external search engine 430.

First, the user terminal 100 may construct a command message based on vehicle information and driver information (S10).

In this regard, the user terminal 100 may primarily determine a vehicle status and a driver status through on-device AI and classify a driver context by a context type. The user terminal 100 may extract necessary data based on the vehicle status and driver status, and generate a command message including the context type and necessary data. In some cases, text data corresponding to voice data may be further included in the command message. The user terminal 100 may summarize and compress the generated command message and transmit the resulting command message to the AI assistant server 300 via a wireless link with the satellite 200.

The AI assistant server 300 that has received the command message may decompress and analyze the command message to identify the context type, which is a type of service requested by the terminal (S20).

The server 300 may acquire specific context information corresponding to the identified context type from the command message (S30). For example, the server 300 may acquire at least one of rescue request information, emergency response information, reservation information, and search information depending on the context type.

The server 300 may operate through interworking with different external interfaces 410, 420, and 430 depending on the acquired information.

In an embodiment, when the context type is identified as 'emergency call' in step S20, the server 300 may acquire rescue request information in step S30 and operate in an emergency call mode (S40). In this case, the server 300 may connect a communication channel with the emergency response center 410 and wake up server-based conversational AI. Through this, the server 300 can convert a rescue request received as text into voice (TTS) and transmit the converted voice to a responder. The server 300 may recognize and process the responder's voice response as text (STT).

In another embodiment, when the context type is identified as 'telephone reservation' in step S20, the server 300 may acquire reservation information including information on reservation date and time and location in step S30, and operate in a telephone reservation mode (S60). In this case, the server 300 may communicate with the service provider 420, such as a restaurant or airline, to perform an automatic reservation call through the server-based conversational AI.

In another embodiment, when the context type is identified as 'information search' in step S20, the server 300 may acquire search information including search keywords in step S30 and operate in an information search mode (S70). In this case, the server 300 may perform information search through interworking with API of the external search engine 430, and extract and summarize only key information, which matches the user's intent, from among search results.

In another embodiment, when the context type is identified as 'emergency response guide' in step S20, the server 300 may acquire emergency response information including patient's symptom information in step S30 and operate in an emergency response guide mode (S50). In this case, the server 300 may perform context analysis (S51) through interworking with an external large-scale language model (LLM 310) to provide additional accurate emergency response guidance. In some embodiments, the server 300 may transmit prompt LLM REQUEST including context information to the LLM 301 and receive an analysis result LLM RESPONSE thereto. Thereafter, the server 300 may restructure the LLM response to generate a step-by-step emergency response manual (S52) and transmit the manual to the user terminal 100.

Finally, the AI assistant server 300 may generate a response message including a final result performed in each of the modes (S40, S50, S60, and S70) and transmit the response message to the user terminal 100 via the satellite 200 (S80). The user terminal 100 which has received the response message may activate a built-in on-device conversational AI function, and provide the received text information to the user 102 by converting the text information into a synthetic voice (TTS) or outputting the text information on a screen.

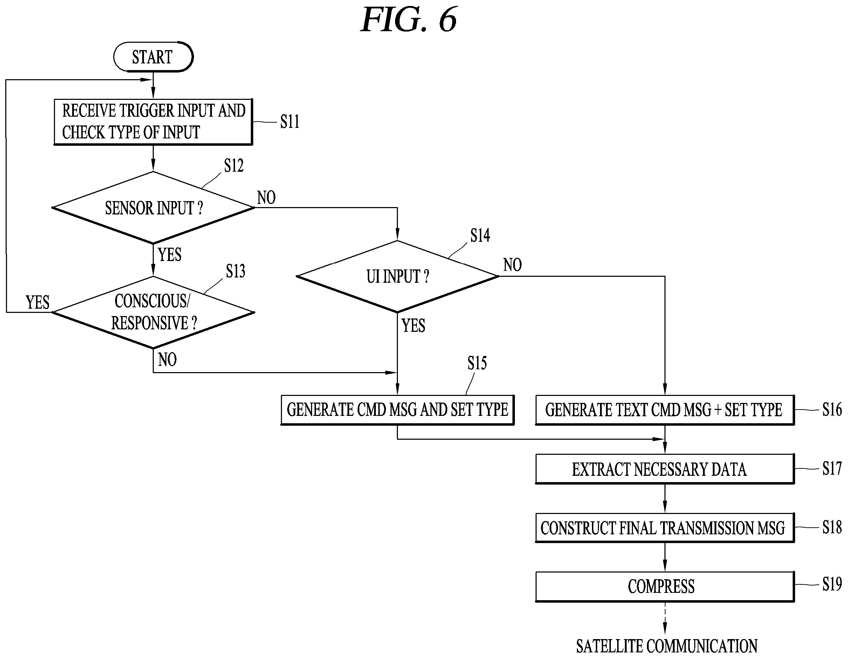

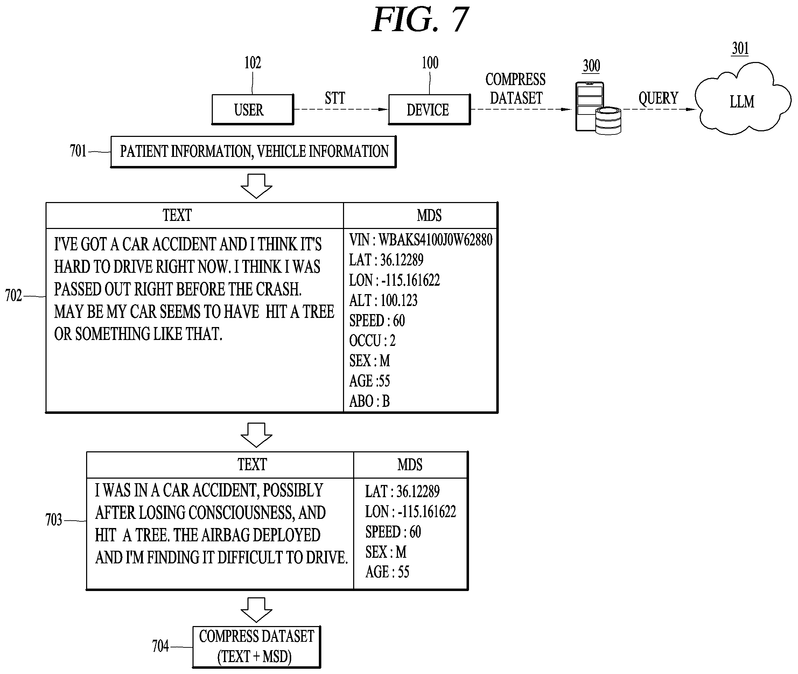

Hereinafter, a command message generation and transmission operation, performed in a user terminal according to the disclosure, will be described. In this regard, FIG. 6 is an operational flowchart of a method of generating and transmitting a command message, performed in a user terminal according to the disclosure. FIG. 7 is a view of data flow for command message generation, summarization, and compression processes performed in a user terminal according to the disclosure.

Referring to FIGS. 1 to 7, a command message generation and transmission operation may be performed by the processor 170 of the user terminal 100.

First, the processor 170 may receive a trigger input from the user terminal 100 and check a type of the input (S11). The trigger input may include at least one of user voice, vehicle sensor data, or UI input. Here, UI input may mean input data generated by the user 102 operating the terminal 100.

In some embodiments, the user's voice may be a natural language-based speech input through an in-vehicle microphone, and may be converted into text through subsequent correction and STT processes. Vehicle sensor data may be acquired through an acceleration sensor, a collision detection sensor, an airbag sensor, a biometric sensor, or a posture recognition sensor, and may reflect vehicle status and driver status. In an embodiment, the vehicle sensor data may further include vehicle surrounding environment information based on surrounding images and noises acquired through cameras or microphones. The UI input may include inputs through an emergency call button, touch screen operation, UI menu selection, or vehicle control panel.

The processor 170 may determine a trigger type based on source and content of the trigger input. In an embodiment, the processor 170 may determine whether the trigger input is a sensor input for priority processing (S12).

In case that the trigger input is a sensor input ('Yes' in S12), the processor 170 may additionally determine the presence or absence of consciousness or response from the driver, based on the driver information (S13). At this time, the processor 170 may detect the driver's movements or moaning sounds by analyzing in-vehicle camera images or microphone inputs using the on-device AI model. When it is determined that the driver's the consciousness or response is present, it may be interpreted that the user or driver can recognize the context. This context may not be an emergency requiring immediate rescue, and accordingly, the processor 170 may go back to step S11 or switch to a standby state to wait for the next trigger input.

In another example, when the consciousness or response is not present ('No' in S13), the processor 170 may classify the driver context as TYPE 0 by determining that the driver is in an emergency context where normal operation or response is impossible. Here, TYPE 0 may mean an 'emergency call' context. The processor 170 may generate a command message corresponding to the classified type and set the generated message to include the corresponding type (S15). In this case, a message including a minimum set of data (MSD) for an emergency rescue request may be generated.

In some embodiments, when it is determined in step S12 that the trigger input is not a sensor input ('No' in S12), the processor 170 may determine whether the input is a result of UI manipulation (S14). When the trigger input is a UI input ('Yes' of S14), the processor 170 may determine that the user intends to make a general service request or information search call. Accordingly, the processor 170 can classify the driver context as TYPE 3 or TYPE 4. Here, TYPE 3 may indicate a 'telephone reservation' context and TYPE 4 may indicate an 'information search' context. Thereafter, the processor 170 may generate a command message corresponding to the classified type and set the generated message to include a corresponding type (S15).

When it is determined in step S14 that the trigger input is not a UI input ('No' in S14), the processor 170 may determine that the trigger input is by a user voice. At this time, the processor 170 may perform correction, such as removing ambient noise, on the input voice data and then perform STT to convert the voice data into text data. The processor 170 may classify the driver context type as one of TYPE 1 or TYPE 2 based on the converted text data and the input context. Here, TYPE 1 and TYPE 2 may indicate 'emergency response guide' contexts. Thereafter, the processor 170 may set the classified type and generate a text-based command message corresponding to the type (S16).

The processor 170 may extract necessary data corresponding to the set context type (S17). Depending on the context type, the necessary data may include various data, required for type-based processing, such as MSD, location information, sensor information, STT-converted text, and reservation information.

The processor 170 may combine the extracted necessary data, the command message, and the context type to construct a final transmission message (S18). Afterwards, to increase transmission efficiency in an NTN environment, the transmission message may be summarized and compressed (S19). At this time, the processor 170 may apply a compression ratio which is determined based on the urgency level of the context type or communication quality. Finally, the compressed transmission message may be transmitted via satellite communication using a satellite communication modem.

In this regard, referring to FIG. 7, the user terminal 100 may acquire various input information 701.

In an embodiment, the input information 701 may be directly acquired in the form of raw data, such as user voice, vehicle identification number (VIN), altitude (alt), number of occupants (occu), blood type (ABO), and the like. In another embodiment, the input information may be vehicle information and driver information acquired by processing through an on-device AI model. For example, patient information may include gender (male), age (55 years old), blood type (B), a semi-conscious state, headache, and chest pain. Vehicle information may include airbag activation, vehicle rollover X, 60 km/h, and the like.

That is, the user terminal 100 may analyze the received voice data, sensor data, and UI input using the on-device AI model, and convert such data into vehicle information and driver information required for contextual awareness, thereby acquiring the vehicle information and driver information.

The user terminal 100 may combine the text data that has undergone STT conversion and initial minimum set of data (MSD) (necessary data) to construct an initial dataset 702. The initial dataset 702 may include all the raw information collected and may be, for example, about 366 bytes in size.

The user terminal 100 may perform a text data summarization and necessary data selection process to maximize message transmission efficiency. Through the process, an optimized dataset 703 may be generated, which may include only essential MSD data for each context and may be reduced to a size of, for example, about 269 bytes.

The user terminal 100 may apply a compression algorithm to the summarized and selected dataset to generate a final compressed dataset (704). The compressed dataset 704 may be generated with a size of, for example, about 217 bytes, and the user terminal 100 may then transmit the compressed dataset 704 to the AI assistant server 300 via the satellite 200.

In some embodiments, the AI assistant server 300 that has received the compressed command message 704 may analyze the received compressed dataset.

First, the server 300 may analyze the received command message and perform contextual awareness corresponding to the driver context. That is, the server may identify a specific context of the driver based on the context type, text data, and necessary data included in the command message, and acquire information required for the context (rescue request information or reservation information).

The server 300 may perform an initial step of selecting an optimal prompt model to query the external LLM 301 based on at least one of the identified context type and the acquired context information. At this time, the external LLM 301 may be implemented as a server (cloud AI server) or platform that provides cloud AI services.

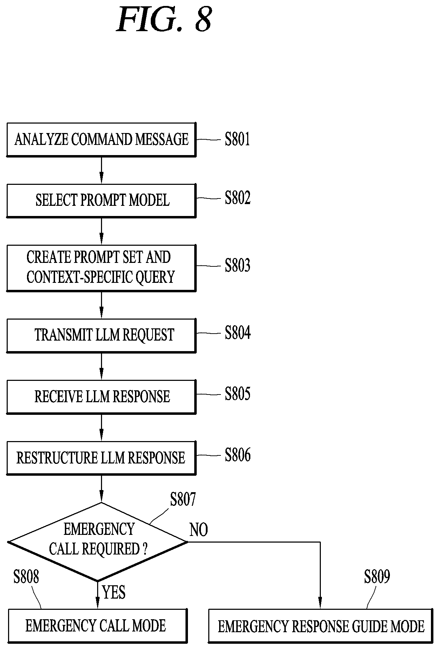

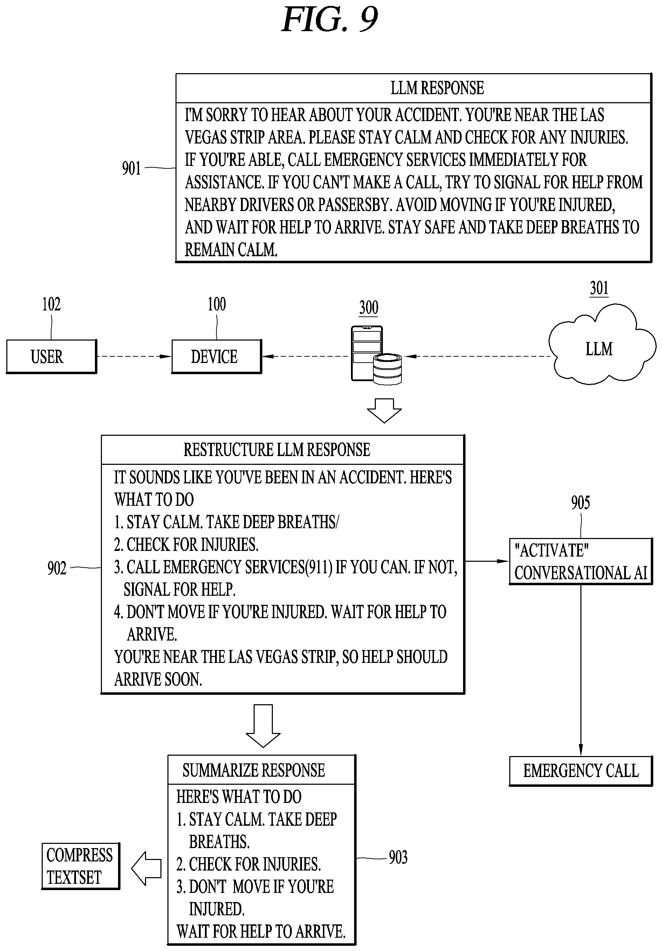

Hereinafter, an LLM linkage-based context analysis method, performed in an AI assistant server according to the disclosure, will be described. In this regard, FIG. 8 is an operational flowchart of an LLM linkage-based context analysis and response mode determination process performed in an AI assistant server according to the disclosure. FIG. 9 is a view of an example of data processing and mode switching in which an AI assistant server according to the disclosure restructures LLM analysis results and provides the restructured analysis results to a user.

Referring to FIGS. 1 to 9, a context analysis and response mode determination process based on LLM linkage may be performed by the processor 370 of the AI assistant server 300.

When the context type is identified as an emergency response guide as a result of the context identification in step S20 of FIG. 5, the processor 370 of the server 300 may perform additional context analysis by interworking with the LLM 301.

To this end, the processor 370 may structure LLM REQUEST requesting for context analysis through interworking with the LLM 301 and transmit the LLM REQUEST to the LLM 301.

In this regard, the processor 370 may first analyze the received command message (S801). Next, the processor 370 may select a context-specific prompt model based on at least one of the context type included in the command message, necessary data, and text data corresponding to the user voice (S802).

In this specification, a prompt model may indicate a predefined query template model configured to enable the LLM 301 to more specifically and accurately determine the driver context, i.e., a template model for generating LLM input. The prompt model may include context-sensitive query structures, context description templates, or response-guiding rules. For example, when the command message is a message notifying an occurrence of an accident, the processor 370 may select a prompt model dedicated to an accident response to analyze the accident context.

In this regard, the prompt model may be implemented as an AI model based on machine learning or deep learning. The prompt model may be pretrained based on various emergency scenarios, stored in a memory of the server 300, and loaded and executed by the processor 370.

In an embodiment, the training process of the prompt model may be performed as follows. First, learning dataset may be constructed as a pair of an abbreviated command message, which is 'input data' and reflects the constraints of a satellite communication environment (e.g., context type code, sensor numerical data, fragmentary voice keywords), and 'optimal natural language query,' which is 'label data (ground truth)' and written by experts so that LLM can understand the corresponding context.

The prompt model may be supervised and trained to generate sentences having a high similarity to the optimal natural language query when the abbreviated command message is input. For example, when given vector data like "Type=0, Impact=High, HR=Unstable" as input, the prompt model may be optimized through weight tuning to expand and generate the input into a context-rich prompt, such as "A high-speed crash has been detected and the driver's heart rate is unstable, indicating a critical emergency that requires immediate medical attention. Please suggest prioritized emergency protocols."

The prompt model may be continuously tuned through reinforcement learning. That is, by setting the quality (accuracy, usefulness) of an answer derived when the generated prompt is input into LLM as a reward function, the prompt model may be configured to learn a prompt structure that elicits better answers from LLM on its own.

The processor 370 may create a prompt set and a contextual query using the selected prompt model (S803). In this regard, the prompt set may be defined as a set of questions including a plurality of sentences or a plurality of queries generated based on the selected prompt model. For example, the prompt set may include a basic prompt, such as "Please determine whether a vehicle accident has occurred and identify the circumstances of the accident." The contextual query may be a supplementary query designed to enable the LLM 301 to perform accurate situational judgment by reflecting necessary input data, such as command messages, sensor data, and MSD. For example, the contextual query may include "An airbag=1 value has been detected. Is the crash at a level that requires emergency response?"

Thereafter, the processor 370 may construct LLM REQUEST including the prompt set and the contextual query and transmit the same to the LLM 301 (S804). The LLM REQUEST may include text corresponding to the user's speech, and in some cases, the LLM REQUEST may further include necessary data. For example, the LLM REQUEST may include text data corresponding to the user's speech, such as "It's hard to move" or "My car hit something." In some cases, the LLM REQUEST may include additional necessary data, such as location information, speed information, and whether the airbags have deployed.

In response to the LLM REQUEST transmission, the processor 370 may receive LLM RESPONSE including a context analysis result from the LLM 301 (S805). In this regard, the LLM RESPONSE may be a natural language-based response generated by the LLM 301 by analyzing user utterance text included in the LLM REQUEST, a set of prompts, context-specific queries, and necessary data. In an embodiment, the LLM RESPONSE may include an assessment of the accident context, a determination of the need for emergency responses, and measures for stabilizing the driver.

For example, as shown in FIG. 9, LLM RESPONSE 901 may include location and situational assessment sentences for the accident context, such as "You're near the Las Vegas Strip area." The LLM RESPONSE 901 may also include instructions for emergency responses and stabilization measures, such as "Please stay calm and check for any injuries" and "Avoid moving if you're injured, and wait for help to arrive."

In some embodiments, the processor 370 may analyze and restructure the received LLM RESPONSE (S806). As shown in FIG. 9, the original LLM RESPONSE 901 may typically include long natural language sentences of several hundred bytes (e.g., approximately 426 bytes). These lengthy responses may not allow for easy extraction of key information needed to determine whether an emergency response is necessary. The lengthy responses may not be suitable for direct transmission in a satellite communication environment, considering transmission efficiency.

Therefore, the processor 370 may remove long natural language responses, redundant information, or unnecessary explanations included in the LLM RESPONSE, extract only the key information necessary for performing emergency responses, and convert the extracted information into a structured form. For example, as shown in FIG. 9, the processor 370 may analyze the contents of the LLM RESPONSE 901 to generate a restructured LLM response 902 (e.g., 360 bytes) including core instructions.

The processor 370 may analyze the restructured LLM RESPONSE to determine whether emergency responses are necessary (S807). That is, the processor 370 may determine whether to switch to the emergency call mode based on the restructured LLM RESPONSE.

For example, as shown in FIG. 9, the restructured LLM RESPONSE 902 may include words or sentences that directly instruct calling external emergency services (e.g., 911), for example, "Call emergency services (911) if you can. If not, signal for help." In this case, the processor 370 may determine that an immediate emergency response is required. On the other hand, when the restructured LLM RESPONSE includes only emergency step-by-step first aid instructions, such as "Avoid moving if you're injured" or "Wait for help to arrive," without direct phrases to call emergency services, the processor 370 may determine that an immediate response is not required and that providing an emergency response manual to the user is appropriate.

In an embodiment, when it is determined that an immediate emergency response is required ('Yes' in S807), the processor 370 may operate in the emergency call mode (S808). At this time, the processor 370 may activate "conversational AI" (905 in FIG. 9) to perform server-based interactive TTS. The processor 370 may be controlled to connect an emergency call to the emergency response center 410 based on the restructured LLM RESPONSE. That is, the processor 370 may connect an emergency call to the emergency response center 410 by activating the server-based conversational AI, and perform voice-based conversations with the emergency response center based on the restructured LLM RESPONSE.

Here, "server-based interactive TTS" may indicate a method in which the server 300 performs emergency response guidance sentence generation, voice synthesis, and conversation flow control. "Server-based interactive TTS" may be a real-time voice interaction method in which the server 300 directly performs voice output or call connection control. Due to this, even in situations where it is difficult for the driver to make a call, the server 300 may immediately initiate voice guidance or perform connection to emergency services on behalf of the driver.

The processor 370 may acquire feedback information including dispatch information and response results of emergency responders. The processor 370 may generate a response message including the feedback information and transmit the response message to the user terminal 100 via the satellite 200.

In another embodiment, when it is determined that an immediate emergency response is not required ('No' in S807), the processor 370 may continue to operate in the emergency response guide mode (S809).

At this time, the processor 370 may analyze the restructured LLM response to acquire a plurality of step-by-step guide texts to be sequentially provided to the user. The processor 370 may generate a response message including the plurality of step-by-step guide texts. Here, the response message may further include an 'on-device interactive TTS command' which activates the on-device AI model of the user terminal 100 to provide a text-based guide in voice.

In an embodiment, the processor 370 may summarize the restructured LLM RESPONSE, taking into account the data transmission efficiency of satellite communication. Through this, the processor 370 may generate a summarized response (see 903 of FIG. 9, e.g., approximately 181 bytes), which is an abbreviation of an original text.

The processor 370 may compress the summarized response 903 again into a binary form to generate a compressed dataset (see 904 of FIG. 9, e.g., approximately 163 bytes) and transmit the generated compressed dataset 904 to the user terminal 100 via the satellite 200.

In response, the processor 170 of the user terminal 100 may restore the received compressed dataset 904 and identify the included TTS command. When the TTS command is identified, the user terminal 100 may immediately operate in an on-device-based interactive TTS. Accordingly, the processor 170 can perform an operation of converting the restored step-by-step guide text into a synthetic voice signal and sequentially guiding the converted synthetic voice signal to the driver through a speaker.

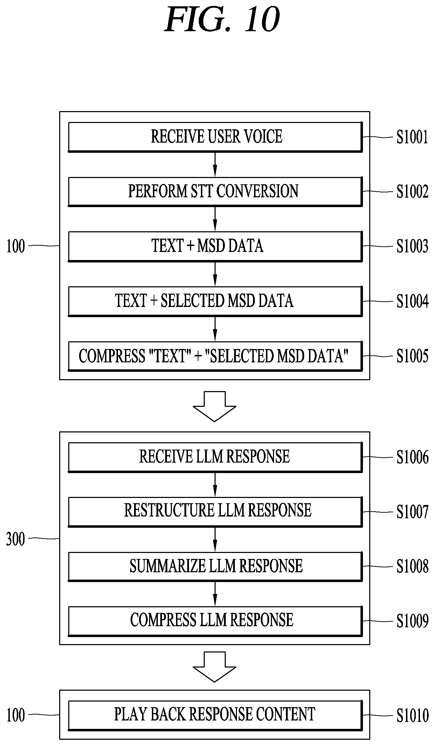

Hereinafter, a description will be given of data restructure, summarization, and compression which are suitable for an NTN-based satellite communication environment performed in a user terminal and an AI assistant server according to the disclosure. In this regard, FIG. 10 is a view of an embodiment of data restructure, summarization, and compression performed in a user terminal and an AI assistant server according to the disclosure.

Referring to FIGS. 1 to 10, the data restructure, summarization, and compression may be performed by each of the processor 170 of the user terminal 100 and the processor 370 of the server 300.

First, the processor 170 of the user terminal 100 may receive voice data including the user's voice (S1001). In an embodiment, the voice data may be configured as data of about 11 seconds long (e.g. approximately 11,000 bytes).

At this time, the voice data may include not only the user voice, but also ambient environmental sounds, such as vehicle driving noise, external crash sounds, tire friction noise, or siren sounds. The included user voice may include unstructured sentences, exclamations, and incomplete sentences uttered in a driving environment or immediately after an accident.

Accordingly, the processor 170 according to the disclosure can perform correction to remove background noise included in the input voice data or increase the clarity of the voice signal. This may be to suppress the voice recognition rate from being reduced due to wind noise, engine noise, or popping noise during a crash, which may occur while driving. The processor 170 may then convert the corrected voice data into text data using an STT model (S1002). The converted text data may be, for example, "text: I've got a car accident and I think it's hard to drive right now. I think I was passed out right before the crash. Maybe my car seems to have hit a tree or something like that." Next, the processor 170 may generate a message in the form of "text + MSD data" by combining the converted text with the overall necessary data (e.g., MSD) (S1003).

That is, the processor 170 may perform adding the converted text data to the command message. At this time, the entire MSD data may correspond to reference numeral 702 of FIG. 7, and may include additional information, such as last stopping point, previous driving history, vehicle internal temperature, tire pressure, and the like, in addition to basic information, such as location coordinates (lat, lon) and vehicle speed. In this case, "text + MSD data" may be about 366 bytes in size.

In an embodiment, the processor 170 may select only several items (e.g., whether the airbags have deployed, location coordinates, speed information, and the like), which are required for accident determination, from the overall MSD data, thereby generating a message in the form of "text + selected MSD data" along with the converted text (S1004). At this time, "text + selected MSD data" may correspond to reference numeral 703 in FIG. 7.

Although not shown, the processor 170 may generate a message in a manner of reducing the size of the message by summarizing the message in the form of "text + MSD data." For example, the processor may perform summarization in a manner of leaving only key sentences by removing unnecessary conjunctions, repetitions, and contextually redundant explanations from the text.

The processor 170 may compress the "text + selected MSD data" based on the summarized text and the selected MSD data (S1005).

In this process, the processor 170 may apply different summarization levels or compression rates to the command message based on at least one of the context type and the satellite communication link quality.

In an embodiment, the processor 170 may apply a first compression ratio and a high-intensity summarization centered on core keywords to minimize data loss in an emergency call or emergency response guide context or when communication quality is low. On the other hand, in a telephone reservation or information search context and when the communication quality is good, the processor 170 may optimize data transmission by applying a second compression ratio that increases transmission efficiency and a low-intensity summarization that maintains context. Through this process, compressed data can be generated with a size of about 217 bytes, and the processor 170 can transmit the compressed data to the AI assistant server 300 via satellite communication.

In some embodiments, the processor 370 of the AI assistant server 300 may receive the compressed message from the terminal 100, decompress the compressed message, and perform subsequent processing according to the context type. For example, in the case of an accident or emergency context classification such as TYPE 1 or TYPE 2, the processor 370 may preferentially perform an LLM-based context judgment procedure.

In this regard, the processor 370 may transmit LLM REQUEST to the LLM 301 and receive corresponding LLM RESPONSE (S1006). In an embodiment, the LLM RESPONSE may correspond to reference numeral 901 of FIG. 9, and may be a natural language text including an accident context assessment and emergency guidance sentences (e.g., approximately 426 bytes).

Next, the processor 370 may restructure the LLM RESPONSE (S1007). For example, the processor 370 may remove redundant sentences or unnecessary explanations from long LLM RESPONSE (e.g. 426 bytes) and select only key instructions necessary for actual emergency response judgment, thereby generating a restructured response (902 in FIG. 9) of approximately 360 bytes in size.

The processor 370 may then summarize the restructured LLM RESPONSE (S1008). In an embodiment, the processor 370 may generate a concisely summarized response (e.g., approximately 181 bytes) by converting emergency guidance sentences into a numbered step-by-step instruction format or summarizing only core action guidelines.

Finally, the processor 370 may recompress the summarized LLM RESPONSE (S1009). In an embodiment, the compressed summarized response may correspond to reference numeral 904 of FIG. 9 and may be generated with a size of approximately 163 bytes. This can ensure transmission efficiency and delay minimization in NTN-based satellite communication environments. The generated summarized and compressed message may be transmitted to the user terminal 100 via the satellite 200.

Finally, the processor 170 of the user terminal 100 may decompress the compressed summarized response received from the server 300 and play back the response using the on-device based interactive TTS (S1010).

In this way, the user terminal 100 and the AI assistant server 300 according to the disclosure can apply an intelligent data processing method that selects only necessary data during the message generation process or summarizes and compresses text appropriately to context, taking into account the transmission efficiency of the NTN-based satellite communication environment.

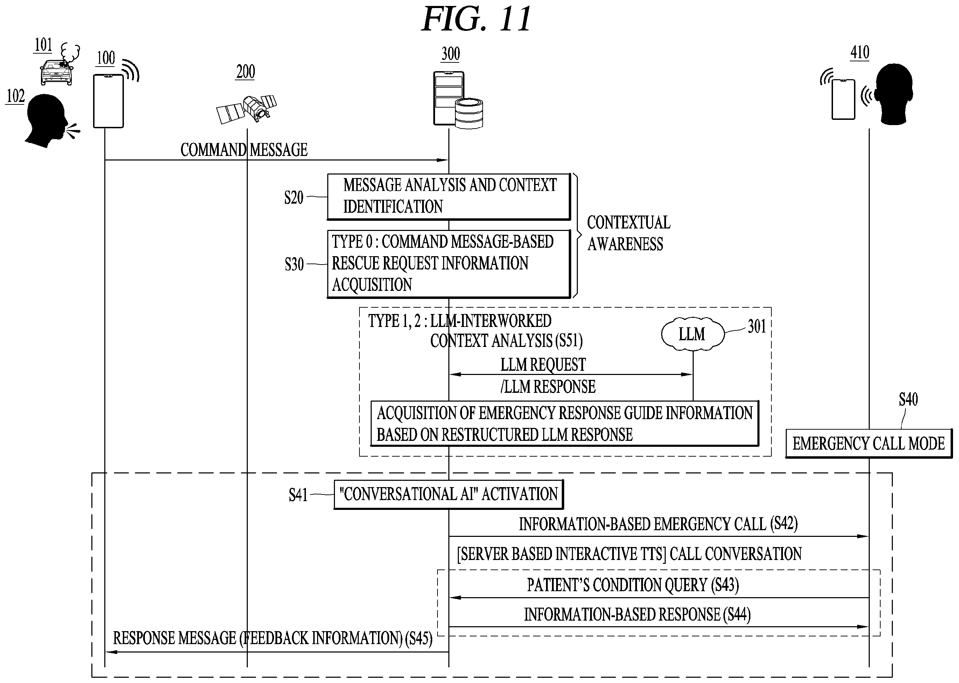

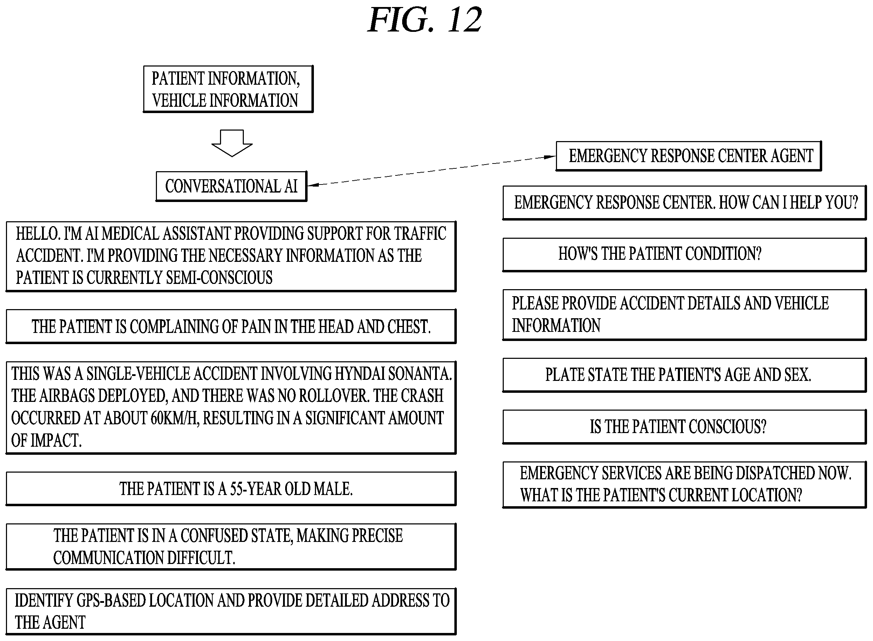

Hereinafter, an operation method in an emergency call mode in a satellite communication system according to the disclosure will be described. In this regard, FIG. 11 is an operational flowchart in an emergency call mode performed by an AI assistant server according to the disclosure. FIG. 12 is a view of an embodiment of server-based interactive TTS conversations performed in an emergency call mode according to the disclosure.

Referring to FIGS. 1 to 12, operations in an emergency call mode in a satellite communication system may be implemented through interconnection among the user terminal 100, the satellite 200, the AI assistant server 300, and the emergency response center 410.

First, the user terminal 100 may transmit a compressed command message generated based on vehicle information and driver information to the server 300 via the satellite 200.

In this regard, the user terminal 100 may generate a trigger signal when detecting an impact from a sensor mounted inside or outside the vehicle. In some embodiments, to exclude noise of sensor values or minor vibrations, the terminal 100 may identify an impact as a valid trigger only when the impact exceeds a pre-learned hysteresis range.

At this time, the terminal 100 may perform a probe (user consciousness probe) procedure to confirm the driver's response. When the driver's voice or touch response is confirmed, the terminal 100 may ignore the trigger, but when there is no response within a preset time (No response), the terminal may judge it as an actual emergency context.

Accordingly, the user terminal 100 can classify the driver status as an 'emergency call (Type 0)' status and generate a command message including corresponding text data. In an embodiment, the text data may be an auto-generated phrase that summarizes the context, such as "[Automated Alert] Crash detected. "User unresponsive."

The user terminal 100 may further include context type, trigger subject information (e.g., sensor), and location information in the command message, compress the command message, and transmit the compressed command message to the server 300.

In some embodiments, the AI assistant server 300 may receive the compressed dataset transmitted from the terminal 100 and decompress the compressed dataset. The processor 370 of the server 300 may perform contextual awareness by analyzing the text data included in the command message and necessary data, and identify the context type (S20).

In an embodiment, when the trigger is determined as a trigger by a sensor according to a result of the analysis, the processor 370 may process the trigger as a top priority and determine that it is an emergency context requiring an emergency rescue request (911 call).

The processor 370 may acquire context information according to the identified context type (S30). In an embodiment, when the identified context type is TYPE 0 (emergency call), the processor 370 may combine the recognized specific accident context and MSD data (location, whether the airbags have deployed, and the like.) to generate rescue request sentences to be delivered to a responder. In the disclosure, the generated rescue request sentences may be acquired as rescue request information.

In this case, the processor 370 may decide to immediately enter the emergency call mode (S40) and connect the 911 call.

On the other hand, in case of TYPE 1 or 2 (emergency response guide), the processor 370 may perform LLM-interworked context analysis for precise context judgment (S51). At this time, the processor 370 may re-evaluate the urgency level of the context by analyzing LLM REQUEST and LLM RESPONSE transmitted and received to and from the LLM 301. When it is determined that an immediate rescue is required based on the restructured RESPONSE, the processor 370 may acquire rescue request information from the RESPONSE and enter the emergency call mode (S40).

Thereafter, the processor 370 may operate in the emergency call mode (S40) based on the acquired rescue request information.

When the emergency call mode (S40) is executed, the processor 370 of the AI assistant server 300 may activate the "conversational AI" module inside the server (S41). That is, the emergency call mode (S40) may refer to an AI-based emergency response system mode. In this regard, the conversational AI may perform a function of converting text data into speech (TTS), converting input speech into text (STT), and managing natural language conversation flow.

The processor 370 may connect the emergency call to the emergency response center 410 based on the acquired information (location, vehicle information, patient status, and the like) (S42).

Once the connection is established, the processor 370 may operate in a server-based interactive TTS mode to perform phone conversations.

In an embodiment, when a query asking about a patient's condition or on-site context is received from the responder of the emergency response center 410 (S43), the processor 370 may recognize this in real time. Thereafter, the processor 370 may generate an appropriate response based on sensor data or LLM analysis results in the acquired command message, convert the generated response into a synthetic voice, and transmit the response to the responder (S44). Through this, even in contexts where the driver loses consciousness or is unable to make a call, the AI assistant server 300 may exchange detailed contextual explanations with the responder on behalf of the driver.

For example, referring to FIG. 12, the processor 370 may analyze the command message or the restructured LLM response in step S30 or step S51 of FIG. 11, to acquire rescue request information including driver information and vehicle information.

In this regard, the processor 370 may acquire patient information and vehicle information indicating the driver's condition and accident details, as shown in FIG. 12.

The processor 370 may activate server-based conversational AI to perform voice conversations with the emergency response center agent based on the acquired information.

At this time, when a question, such as "What is the patient's condition?" is received from the agent of the emergency response center 410, the processor 370 may generate and transmit a natural language response, such as "The patient is a 55-year-old male. He's currently unconscious and complaining of pain in head and chest," based on the acquired driver information.

When an additional query "Please tell me the details of the accident and vehicle information" is received from the agent, the processor 370 may transmit detailed information by voice, such as: "This is a single-vehicle accident involving Hyundai Sonata. The airbags have deployed, and no rollover has been detected. Crash has occurred at 60 km/h," based on the vehicle information.

Finally, when the agent asks for a location for dispatching an ambulance, the processor 370 may support rapid dispatch by providing the agent with accurate latitude/longitude information based on GPS or address information converted from the latitude/longitude.

Finally, the processor 370 may generate feedback information (S45) as a result of the emergency call and construct a response message including the feedback information. In this regard, feedback information may include emergency responder dispatch information and response results. In an embodiment, the processor 370 may summarize and compress the response message by considering the satellite communication bandwidth and then transmit the resulting response message to the user terminal 100 via the satellite 200.

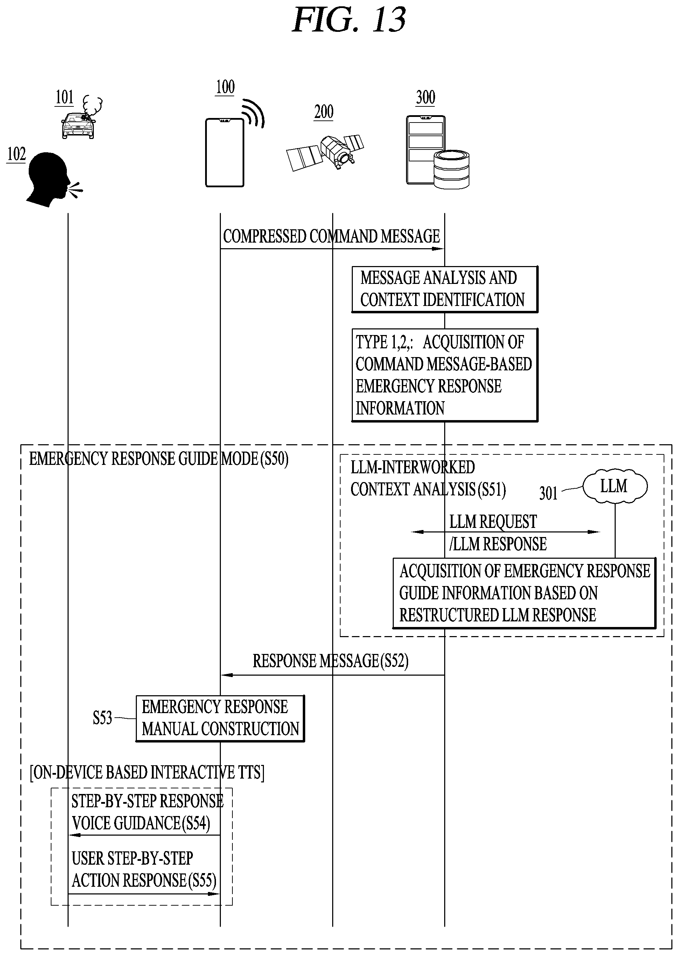

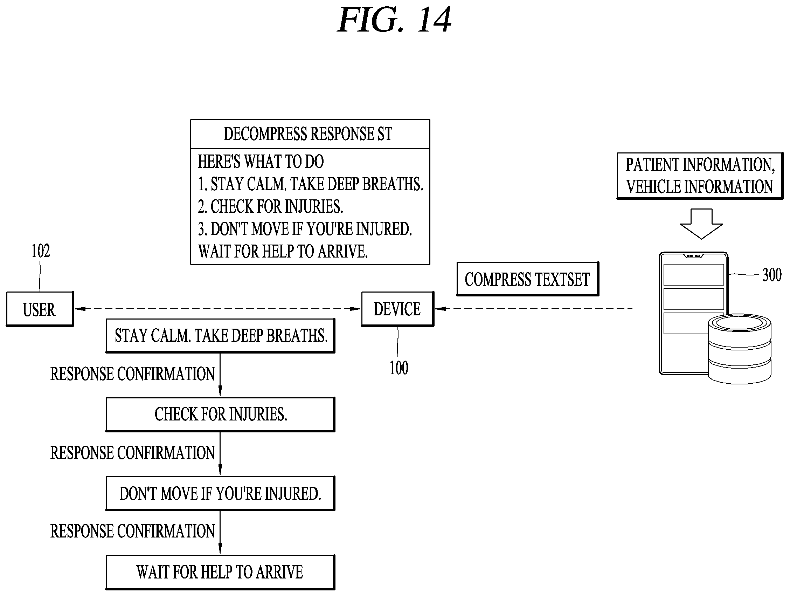

Hereinafter, an operation method in an emergency response guide mode in a satellite communication system according to the disclosure will be described. In this regard, FIG. 13 is an operational flowchart in an emergency response guide mode performed by an AI assistant server according to the disclosure. FIG. 14 is a view of an embodiment of on-device based interactive TTS conversations performed in an emergency response guide mode according to the disclosure.

Referring to FIGS. 1 to 14, operations in the emergency response guide mode in a satellite communication system may be implemented through interconnection among the user terminal 100, the satellite 200, and the AI assistant server 300.

First, the user terminal 100 may transmit a compressed command message, which is generated based on vehicle information and driver information, to the server 300 via the satellite 200.

In this regard, the user terminal 100 may receive a user voice through a microphone. For example, when the user provides an unclear utterance, such as "I had an accident... uh... I don't know what to do," the terminal 100 may detect the utterance and perform the STT function to convert the user voice into text data.

At this time, the user terminal 100 may identify the user's core intent from the converted text data using the on-device AI model and correct the text based on the recognized user's intent. For example, the AI model may analyze rambling utterances and refine the utterances into sentences with a clear meaning: "Reporting an accident and requesting an emergency response guidance."

The user terminal 100 may extract necessary data associated with the corrected text data (e.g., vehicle location, speed, and the like), include the extracted necessary data into a command message, compress the command message, and transmit the compressed command message to the server 300.

In some embodiments, the AI assistant server 300 may receive the compressed dataset transmitted from the terminal 100 and decompress the compressed dataset. The processor 370 of the server 300 may perform contextual awareness by analyzing the text data included in the command message and necessary data, and identify the context type (S20).

The processor 370 may acquire context information according to the identified context type (S30). In an embodiment, when the identified context type is 'emergency response guide (TYPE 1 or 2),' the processor 370 may analyze the command message and acquire basic emergency response information (S30).

Thereafter, the processor 370 may perform contextual analysis (S51) through interworking with the LLM 301 to provide accurate accident context analysis and emergency response guide. In some embodiments, the processor 370 may transmit LLM REQUEST including context information to the LLM 301 and receive LLM RESPONSE corresponding thereto. The processor 370 may analyze the received LLM RESPONSE and extract and restructure only core step-by-step instructions required for emergency responses.

In some embodiments, even when a trigger is by the user's voice and a context type is transmitted by being set to 'emergency response guide,' the processor 370 may re-evaluate the urgency of the context based on the restructured LLM RESPONSE. At this time, when it is determined that an immediate professional intervention is required (determined that an emergency response is required), the processor 370 may automatically switch from the emergency response guide mode to the emergency call mode (S40).

The processor 370 may generate a response message (S52) based on the restructured LLM RESPONSE and transmit the response message to the user terminal 100 via the satellite 200. At this time, the processor 370 may summarize and compress the response message and transmit the resulting response message to maximize transmission efficiency. The response message may further include an on-device interactive TTS command which activates the on-device AI model and instructs the on-device AI model to guide a plurality of step-by-step guide texts by voice. In an embodiment, the command may be implemented in a manner of including a field in the response message to set 'Interactive TTS mode (True/False)'. For example, the server may transmit the response message by setting the corresponding field to 'True' when the emergency response guide is necessary.

The user terminal 100 may construct emergency response manuals (S53) by receiving the response message from the server 300, decompressing the received response message, and analyzing the decompressed response message. At this time, when the interactive TTS mode is set to 'True' in the response message, the processor 170 of the user terminal 100 may operate in the on-device-based interactive TTS mode.

Here, the "on-device based interactive TTS mode" may mean a method in which the terminal 100 converts text into voice on its own and detects the user's response to proceed with guidance for the next step without real-time communication with the server. This is to provide seamless guidance without being affected by the latency of satellite communication.

In some embodiments, the processor 170 may convert a first guide text, among the plurality of step-by-step guide texts included in the response message, into voice and output the converted voice (S54). The processor 170 may detect the user's response input to the output voice (S55). At this time, when the response input is detected, the processor 170 may sequentially perform an operation of converting a second guide text into voice and outputting the converted voice. Here, the response input may include the user's voice confirmation (e.g., "Yes" or "Next") or a button input on the terminal.

In this regard, referring to FIG. 14, the server 300 may analyze a context of a 55-year-old male driver experiencing clouded consciousness and chest pain, and transmits a compressed text set to the terminal 100, including step-by-step instructions, such as: "1. Stay calm and take a deep breath. 2 Check for injuries. 3 Don't move if you are injured. 4 "Wait for help to arrive."

The user terminal 100 may receive and decompress the compressed text set, and output the first guide text, "Stay calm. Take a deep breath." by voice. Afterwards, when a response input, such as the user taking a deep breath or pressing a confirmation button, is detected, the terminal 100 may immediately provide the second guide text "Check for injuries" by voice.

Through this process, the user can be guided step-by-step safety rules, such as "Don't move if you're injured" and "Wait for help to arrive," under the guidance of the terminal 100 even in contexts where the communication connection is unstable.

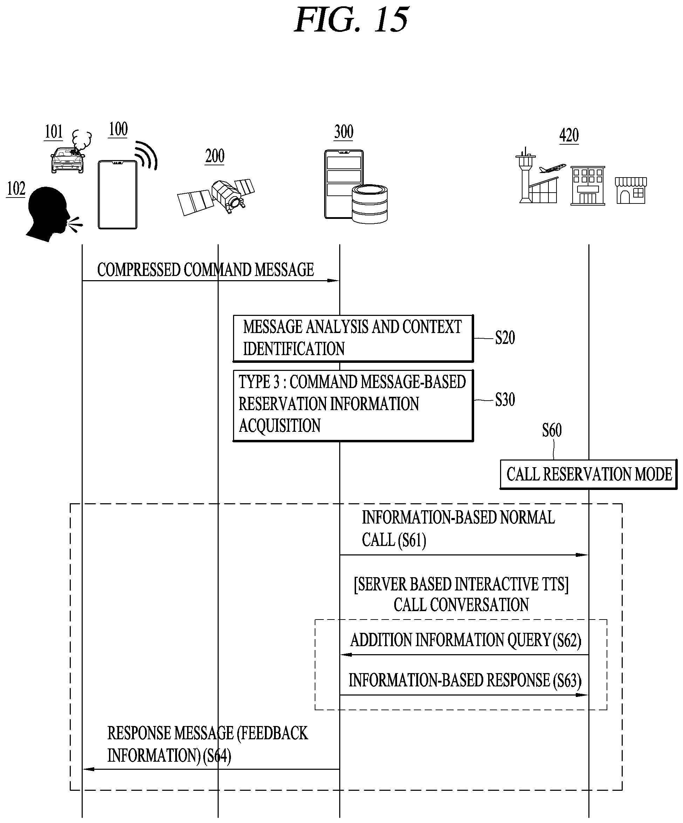

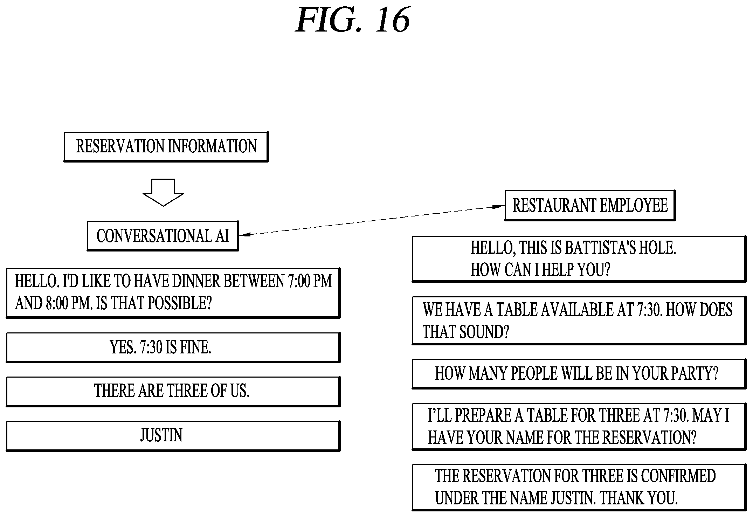

Hereinafter, an operation method in a telephone reservation mode in a satellite communication system according to the disclosure will be described. In this regard, FIG. 15 is an operational flowchart in a telephone reservation mode performed by an AI assistant server according to the disclosure. FIG. 16 is a view of an embodiment of server-based interactive TTS conversations performed in a telephone reservation mode according to the disclosure.

Referring to FIGS. 1 to 16, operations in a telephone reservation mode in a satellite communication system may be implemented through interconnection among the user terminal 100, the satellite 200, the AI assistant server 300, and the service provider 420.

First, the user terminal 100 may transmit a compressed command message generated based on vehicle information and driver information to the server 300 via the satellite 200.

In this regard, the user terminal 100 may receive a trigger signal including a user voice or UI input. For example, the user may say, "Make a reservation for three people at a nearby Italian restaurant in one hour," or may select a reservation menu via a touchscreen of the terminal.

At this time, the user terminal 100 may extract reservation request information (Req Info) from the input and add the extracted reservation request information as necessary data of a command message. Here, the reservation request information may include the name of a person making the reservation (e.g., Justin), number of people (e.g., 3 people), desired time, and location type. The user terminal 100 may compress the generated command message and transmit the command message to the server 300.

In some embodiments, the AI assistant server 300 may receive the compressed dataset transmitted from the terminal 100 and decompress the compressed dataset. The processor 370 of the server 300 may perform contextual awareness by analyzing the text data included in the command message and necessary data, and identify the context type (S20).

The processor 370 may acquire context information according to the identified context type (S30). In an embodiment, when the identified context type is 'telephone reservation (TYPE 3)', the processor 370 may acquire telephone reservation information by analyzing the command message (S30). For example, the reservation information may include reservation type (restaurant, hotel), name of the person making the reservation, number of people, preferred cuisine type, and distance-based conditions (e.g., (reachable within one hour).

In some embodiments, in this embodiment, service targets in TYPE 3 (telephone reservation mode) are not limited to restaurants. For example, service targets may include not only general service providers, such as hotels, airlines, and rental car companies, but also various entities requiring reservations or reception, such as disaster centers, hospital reception desks, or public institutions, depending on contexts.

Thereafter, the processor 370 may operate in the telephone reservation mode (S60) based on the acquired reservation information.

First, the processor 370 may activate a "conversational AI" module inside the server. The processor 370 may activate the server-based conversational AI to connect a call (S61) with the reservation target (service provider 420) included in the reservation information. In an embodiment, the processor 370 may search for a restaurant which meets conditions by considering the user's current location and movement path, and connect a call to the restaurant.

Once the connection is established, the processor 370 may operate in the server-based interactive TTS mode to perform real-time conversations with the reservation target (e.g., restaurant employee).

In this regard, referring to FIG. 16, the processor 370 may analyze the received command message to acquire reservation information necessary for reservation execution. In an embodiment, the reservation information may include at least one of reservation type (e.g., restaurant, hotel), name of the person making the reservation (e.g., Justin), number of people (e.g., 3 people), preferred menu (e.g., Italian, steak), and location-based travel time (e.g., arrival within one hour).

At this time, the processor 370 may recognize the other party's speech and generate a response appropriate to the context based on the acquired reservation information. In some embodiments, upon receiving an inquiry, such as "How can I help you?", from a restaurant employee, the processor 370 may generate a natural language query in real time, such as "Hello. This is Justin's party of three. We are inquiring if dining is available between 19:00 and 20:00," and transmit the generated query via synthetic voice.

When a following inquiry, such as "19:30 is possible. How many people in your party?", is received from the employee (S62), the processor 370 may generate and transmit an information-based response, such as "19:30 is fine. There are three in my family," based on pre-secured information (S63).

Finally, when the employee asks for the name of the person making the reservation, the processor 370 may confirm the reservation by answering, "Justin."

Finally, the processor 370 may analyze call results to generate feedback information which includes whether the reservation has been successful and reservation details. The processor 370 may generate a response message (S64) including the feedback information and transmit the response message to the user terminal 100 via the satellite 200. The user terminal 100 may display information on reservation-confirmed location (address), time, and number of people on a screen or through voice guidance, based on the received response message. The processor 370 may automatically set, if necessary, the reservation location as a destination for a navigation.

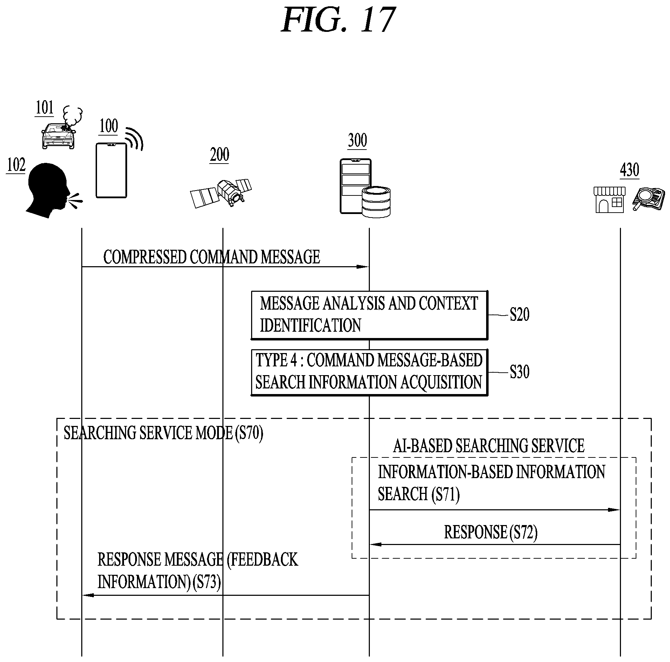

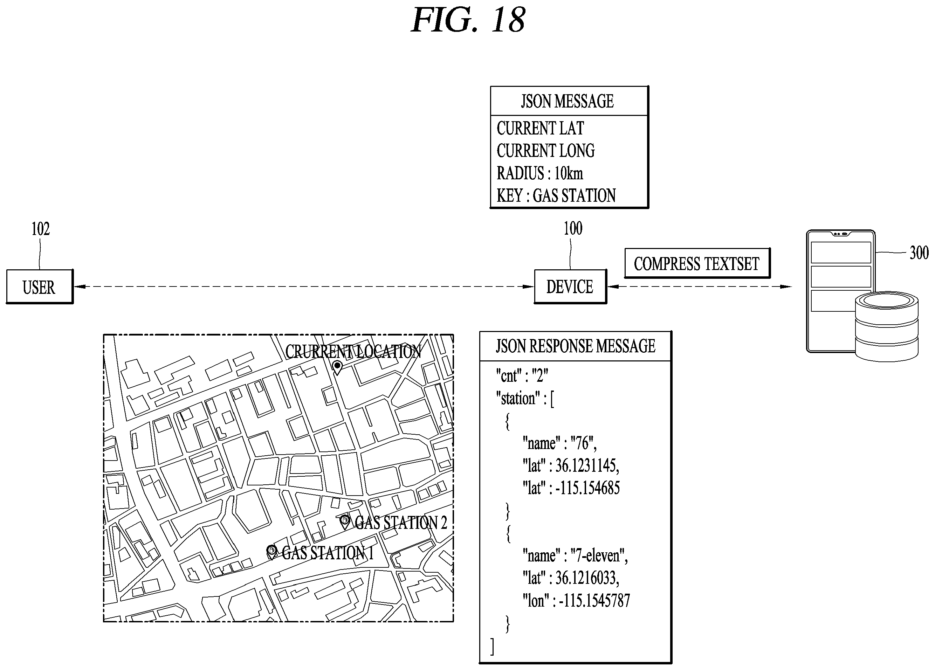

Hereinafter, an operation method in information search mode in a satellite communication system according to the disclosure will be described. In this regard, FIG. 17 is an operational flowchart in an information search mode performed by an AI assistant server according to the disclosure. FIG. 18 is a view of an embodiment of a server-based search method and a search result display method performed in an information search mode according to the disclosure.

Referring to FIGS. 1 to 18, operations in an emergency response guide mode in a satellite communication system may be implemented through interconnection among the user terminal 100, the satellite 200, the AI assistant server 300, and an external search engine 430.

First, the user terminal 100 may transmit a compressed command message generated based on vehicle information and driver information to the server 300 via the satellite 200.

The processor 370 of the server 300 may analyze the received message to identify a context type (S20). The processor 370 may acquire context information according to the identified context type (S30).

In an embodiment, when the identified context type is TYPE 4 (information search), the processor 370 may analyze the command message to acquire search information. The search information may include user-desired search keywords (e.g., gas stations, repair shops, and popular restaurants), search criteria (within a 10km radius and rating of 4.0 or higher), and current location information.

Thereafter, the processor 370 may operate in an information search mode (S70) based on the acquired search information. In some embodiments, the processor 370 may generate a search query based on the acquired search information and perform information search through interworking with the external search engine 430 (S71). That is, the processor 370 may generate a query, such as "gas stations within 10 km at the current location (latitude/longitude)," and transmit the query to map APIs, such as Google or Naver.

When the search is completed, the processor 370 may receive a search result RESPONSE from the search engine 430 (S72). At this time, original search results may include dozens of lists and unnecessary metadata, which may be unsuitable to be transmitted via satellite communication. Accordingly, the server 370 can extract key information that matches the user's intent from result data of the information search, thereby performing text summarization. For example, the processor 370 may lightweight data by selecting only top two best locations in order of distance or rating from the search results and leaving only essential information, such as business name, location, and phone number.

In this regard, referring to FIG. 18, when the user requests "Search for a gas station," the server 300 may acquire information on "Current lat/long (current location)" and "Radius: 10 km (radius)," and "Key: gas station (keyword)" from a message in JSON format. The server 300 may perform an Internet search to acquire information on a plurality of gas stations, and then filter the information to extract only information on two closest gas stations (Gas station 1 and Gas station 2).

Finally, the processor 370 may generate a response message (feedback information) including summarized text data (S73) and transmit the response message to the user terminal 100 via the satellite 200.

As shown in FIG. 18, the server 300 may finally transmit a structured and compressed JSON-format response message (compressed textset), such as "cnt: 2 (number)," "station: [{name: 76, lat:..., lon:...}, {name: 7-Eleven...}]."

In response, the user terminal 100 may parse the received message and display the locations of the searched gas stations as icons on a map. Depending on the user's input, the user terminal 100 may provide functions to display detailed information on a specific location in a pop-up manner or to perform route guidance by immediately setting the location as a destination for navigation.

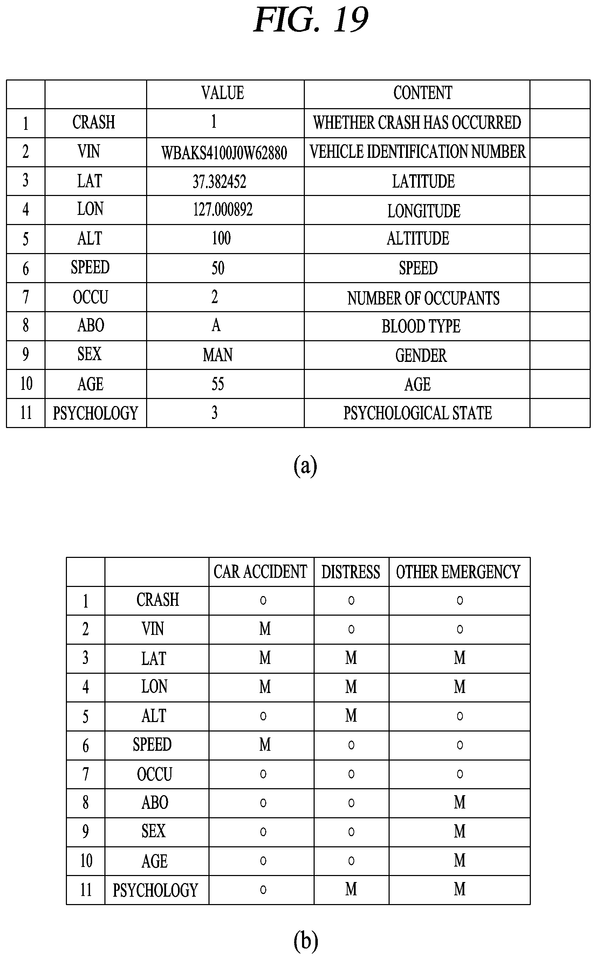

Hereinafter, a method of constructing and extracting necessary data for each context type according to the disclosure will be described. In this regard, FIG. 19 is a view of an embodiment of a minimum set of data (MSD) and a dataset that are selectively configured depending on a context type according to the disclosure.

Referring to FIGS. 1 to 19, the processor 170 of the user terminal 100 may extract data optimized for a classified context type and configure a command message.

In an embodiment, the necessary data may be configured to include different items depending on the context type.

For example, when the context type is 'emergency call (TYPE 0)' or 'emergency response guide (TYPE 1, 2)', the processor 170 may extract information essential for accident handling and rescue as necessary data. In this case, the necessary data may include at least one of whether a crash has occurred (crash), vehicle identification information (vin), location information (lat, lon, alt), vehicle speed (speed), and occupant information (occu, ABO, sex, age). On the other hand, when the context type is 'telephone reservation (TYPE 3)' or 'information search (TYPE 4)', the processor 170 may extract information necessary for performing the service as necessary data. In this case, the necessary data may include at least one of location information, user information (such as name of person making a reservation), vehicle information, and service request information (reservation time and search keywords).

In this regard, referring to FIG. 19, the processor 170 may filter data based on a context-specific essential item table. For example, in case of a 'traffic accident' context, whether a crash has occurred (crash), vehicle identification number (vin), location (lat, lon), and speed (speed) may be key elements for accident analysis, so such information may be designated as mandatory items ('O') and must be included in the message. On the other hand, in case of a 'distress' context, the vehicle's speed information may be relatively less important and may be omitted or treated as an optional item ('M'). Instead, information, such as sex, age, and blood type (ABO) may be essential for identifying the person to be rescued and for providing medical care.

The necessary data according to the disclosure may further include information on the driver's psychological state (psychology). As shown in FIG. 19, the processor 170 may include a psychology index (e.g., value 3 - fear/panic state), which is obtained through voice analysis, in the necessary data. This can allow the rescue center to determine an appropriate level of response by comprehensively assessing not only the physical crash information but also the emotional distress of the driver.

In this way, the processor 170 does not unconditionally transmit overall collected data, but can select and transmit only the necessary data through the context-specific filtering logic defined as in FIG. 19, thereby reducing satellite communication costs and efficiently increasing a data transmission success rate.

In some embodiments, the satellite communication method performed by the user terminal and AI assistant server according to another aspect of the disclosure is not limited thereto, and may be applied in various ways in combination with the embodiments of FIGS. 1 to 19.

So far, a satellite communication system based on vehicle and driver information and an operation method thereof according to this specification have been described. The technical effects of the satellite communication system and the operation method thereof according to this specification will be summarized as follows, but are not limited thereto.

According to the disclosure, by utilizing on-device AI within a terminal in a non-terrestrial network (NTN) environment to convert voice data into text (STT), and selectively extracting, compressing, and transmitting only necessary data according to a context type, the high cost issue and bandwidth constraints of the related art satellite communication can be effectively solved. Accordingly, communication charges can be reduced by minimizing the size of data packets, and data transmission success rate and speed can be dramatically improved even in network congestion situations.

According to the disclosure, unlike the related art methods of simply transmitting text or sensor values, the driver's voice features (tone, intensity, and the like), psychological state, and location information can be comprehensively analyzed through the on-device AI model, thereby enabling precise judgment of whether an accident has occurred and an urgency level. This can reduce false reports and accurately recognize actual emergency contexts, laying the foundation for rescue centers to carry out optimal initial responses within the golden time.

According to the disclosure, even in a text-based satellite communication environment, through interworking between server-based conversational AI and a large language model (LLM), contextually customized emergency response guides can be provided to users or voice calls can be connected with emergency centers on behalf of the users. Accordingly, seamless bidirectional communication can be performed even in contexts where drivers have difficulty making direct calls, and the automatic translation and information search functions can significantly expand user experience (UX) to overseas travel and general convenience service areas.

The disclosure may be implemented as computer-readable codes in a program-recorded medium. The computer-readable media may include all kinds of recording devices in which data readable by a computer system is stored. Examples of such computer-readable media may include hard disk drive (HDD), solid status disk (SSD), silicon disk drive (SDD), ROM, RAM, CD-ROM, magnetic tape, floppy disk, optical data storage element, and the like, and the computer-readable media may also configure non-transitory computer-readable storage media. Also, the computer-readable medium may also be implemented as a format of carrier wave (e.g., transmission via an Internet), but it should be interpreted that the computer-readable medium described in the claims of the disclosure means the non-transitory storage media. The computer may include the controller of the terminal. Therefore, the detailed description should not be limitedly construed in all of the aspects, and should be understood to be illustrative. The scope of the disclosure should be determined by reasonable interpretation of the appended claims and all changes that come within the equivalent scope of the disclosure are included in the scope of the disclosure.

## Claims

1. A satellite communication method comprising:
acquiring at least one of vehicle information and driver information based on at least one of collected voice data, sensor data, and user input data;
classifying a driver context as at least one of a plurality of context types based on the acquired information;
generating a command message based on the context type and the acquired information;
performing data lightweighting to reduce a size of the command message; and
transmitting the lightweighted command message via a satellite.

2. The satellite communication method of claim 1, further comprising:
extracting necessary data associated with the context type; and
adding the necessary data to the command message.

3. The satellite communication method of claim 1, further comprising:
determining whether an emergency context exists or an emergency urgency level based on the acquired information,
wherein the context type is classified based on whether the emergency context exists or the emergency urgency level.

4. The satellite communication method of claim 3, wherein
the context type comprises at least one of emergency call, emergency response guide, telephone reservation, and information search.

5. The satellite communication method of claim 3, wherein
the determining the emergency urgency level comprises:
analyzing acoustic characteristics of the voice data to derive a driver's psychology index; and
determining the emergency urgency level by reflecting the driver's psychology index.

6. The satellite communication method of claim 3, wherein
the determining the emergency urgency level comprises:
acquiring location information from the sensor data to calculate a risk weight corresponding to the location information; and
determining the emergency urgency level by reflecting the risk weight.

7. The satellite communication method of claim 1, further comprising:
converting the voice data into text data using a speech-to-text (STT) model; and
adding the text data to the command message.

8. The satellite communication method of claim 7, further comprising:
determining whether the text data and the sensor data are different from each other; and
performing content correction on the text data by applying priority to the sensor data when the text data and the sensor data are different from each other.

9. The satellite communication method of claim 1, further comprising:
receiving a response message to the transmitted command message from the server via the satellite; and
performing a response operation associated with the received response message.

10. The satellite communication method of claim 2, wherein
the necessary data comprises at least one of where a crash has occurred, vehicle identification information, location information, vehicle speed, and occupant information when the context type is an emergency call or emergency response guide, and
comprises at least one of location information, user information, vehicle information, and service request information when the context type is a telephone reservation or information search.

11. The satellite communication method of claim 1, wherein
the performing the data lightweighting comprises performing summarization or compression on the command message, and
a summarization level or compression rate for the command message is applied differently depending on at least one of the context type and satellite communication link quality.

12. The satellite communication method of claim 11, wherein
the command message is compressed at a first compression ratio when the context type is an emergency call or emergency response guide or when the satellite communication link quality is below a threshold, and
the command message is compressed at a second compression rate lower than the first compression rate when the context type is a telephone reservation or information search and the satellite communication link quality is higher than or equal to the threshold.

13. The satellite communication method of claim 9, further comprising:
decompressing the received command message by a server which has received the command message;
analyzing the command message to identify the context type included in the command message; and
summarizing or compressing a response message including a result of performing a context-specific operation according to the identified context type and transmitting the summarized or compressed response message to a user terminal via the satellite.

14. The satellite communication method of claim 13, further comprising, when the identified context type is an emergency call:
analyzing the command message to acquire rescue request information;
connecting an emergency call with an emergency response center by activating server-based interactive artificial intelligence (AI);
performing a voice-based conversation (call conversation) with the emergency response center based on the rescue request information; and
generating the response message to include feedback information comprising dispatch information or response results of an emergency responder.

15. The satellite communication method of claim 13, further comprising, when the identified context type is an emergency response guide:
structuring a large language model (LLM) request (LLM REQUEST) requesting a context analysis and transmitting the LLM request to the LLM;
in response to the transmission, receiving an LLM response (LLM RESPONSE) comprising a context analysis result from the LLM; and
analyzing and restructuring the LLM response.

16. The satellite communication method of claim 13, further comprising, when the identified context type is a telephone reservation:
analyzing the command message to acquire reservation information;
activating server-based conversational AI to connect a call with a reservation target included in the reservation information;
performing a voice-based conversation with the reservation target based on the reservation information; and
generating the response message to include feedback information including whether the reservation has been successful or reservation details.

17. The satellite communication method of claim 13, further comprising, when the identified context type is information search:
acquiring search information by analyzing the command message;
generating a search query based on the search information and performing an information search by interworking with an external search engine;
performing text summarization by extracting key information that matches a user intent from result data of the information search; and
generating the response message to include the summarized text data.

18. The satellite communication method of claim 15, wherein
the structuring the LLM request requesting the context analysis comprises:
selecting a context-specific prompt model based on at least one of the context type included in the command message, necessary data, and text data;
creating a prompt set or context-specific query using the selected prompt model; and
structuring an LLM request including the prompt set or the context-specific query.

19. The satellite communication method of claim 15, further comprising:
acquiring a plurality of step-by-step guide texts based on the restructured LLM response; and
generating a response message including the plurality of step-by-step guide texts,
wherein the response message further comprises a text-to-speech (TTS) command that activates an on-device artificial intelligence (AI) model to instruct the plurality of step-by-step guide texts via voice.

20. The satellite communication method of claim 15, further comprising:
re-determining whether the driver context is an emergency call context based on the restructured LLM response,
wherein the method further comprises, when the driver context is determined as the emergency call context according to a result of the re-determination:
connecting an emergency call with an emergency response center by activating server-based conversational AI;
performing a voice-based conversation with the emergency response center based on the restructured LLM response; and
generating the response message to include feedback information comprising dispatch information or response results of an emergency responder.

21. The satellite communication method of claim 9, further comprising, in the performing the response operation, when the response message includes a TTS command:
converting a first guide text, among the plurality of step-by-step guide texts included in the response message, into voice and outputting the converted voice,
detecting the user's response input to the output voice, and
sequentially performing an operation of converting and outputting a second guide text into voice when the response input is detected.

22. The satellite communication method of claim 9, wherein
the performing the response operation comprises:
identifying a country code by acquiring at least one of location information and phone number information;
determining a target language based on the identified country code; and
automatically translating the received response message into the determined target language and outputting the translated response message.

23. A vehicle configured to perform satellite communication comprising:
a communication module configured to transmit and receive data to and from a server by interworking with a non-terrestrial network (NTN) via a satellite; and
a processor operably connected to the communication module,
wherein the processor acquires at least one of vehicle information and driver information based on at least one of collected voice data, sensor data, and user input data,
classifies a driver context as at least one of a plurality of context types based on the acquired information,
generates a command message based on the context type and the acquired information,
performs data lightweighting to reduce a size of the command message, and
transmits the lightweighted command message via the satellite.

24. A computer-readable storage medium, storing at least one computer program including instructions that, when executed by at least one processor, cause the at least one processor to perform operations,
wherein the operations comprises: acquiring at least one of vehicle information and driver information based on at least one of collected voice data, sensor data, and user input data,
classifying a driver context as at least one of a plurality of context types based on the acquired information,
generating a command message based on the context type and the acquired information,
performing data lightweighting to reduce a size of the command message, and
transmitting the lightweighted command message via a satellite.
